# EUROPEAN PATENT SPECIFICATION

(11) **EP 3 466 382 B1**
(45) Date of publication and mention of the grant of the patent: **01.07.2020**
(21) Application number: 16904554.9
(22) Date of filing: 06.06.2016
(51) Int. Cl.: B26D 1/36, B26D 1/40, A61F 13/15, B65H 39/14

(54) **PRODUCTION METHOD AND PRODUCTION DEVICE FOR CONTINUOUS SHEET COMPOSITE FOR ABSORBENT ARTICLE**
HERSTELLUNGSVERFAHREN UND HERSTELLUNGSVORRICHTUNG FÜR ENDLOSFOLIENVERBUND FÜR SAUGFÄHIGEN ARTIKEL
PROCÉDÉ DE PRODUCTION ET DISPOSITIF DE PRODUCTION DE COMPOSITE EN FEUILLE CONTINU POUR ARTICLE ABSORBANT

(43) Date of publication of application: 10.04.2019
(73) Proprietor: Unicharm Corporation, Shikokuchuo-shi, Ehime 799-0111 (JP)
(72) Inventor: OBA, Kenji, Kanonji-shi Kagawa 769-1602 (JP); NAKANO, Takumi, Kanonji-shi Kagawa 769-1602 (JP)
(74) Representative: Staeger & Sperling Partnerschaftsgesellschaft mbB
(86) International application number: PCT/JP2016/066736
(87) International publication number: WO 2017/212519

(56) References cited:
- JP-A- 2001 039 615
- JP-A- 2004 305 471
- JP-A- 2011 083 547
- JP-B2- 4 747 103

## Description

### Technical Field

The present invention relates to a method for manufacturing a composite body of continuous sheets pertaining to an absorbent article such as a disposable diaper, and also relates to an apparatus for manufacturing the same.

### Background Art

Conventionally, in a manufacturing line for an absorbent article such as a disposable diaper, a first continuous sheet is cut to produce a cutform sheet that has a predetermined length, and the produced cutform sheet is bonded and transferred to a second continuous sheet while forming an interval on the second continuous sheet between that cutform sheet and another cutform sheet adjacent thereto in the continuity direction of the second continuous sheet.

### Citation List

### Patent Document

[Patent Document 1] JP 2007-260875A.
Further prior art in this technical field is disclosed in document JP 2004 305471 A.

### Summary of Invention

### Technical Problem

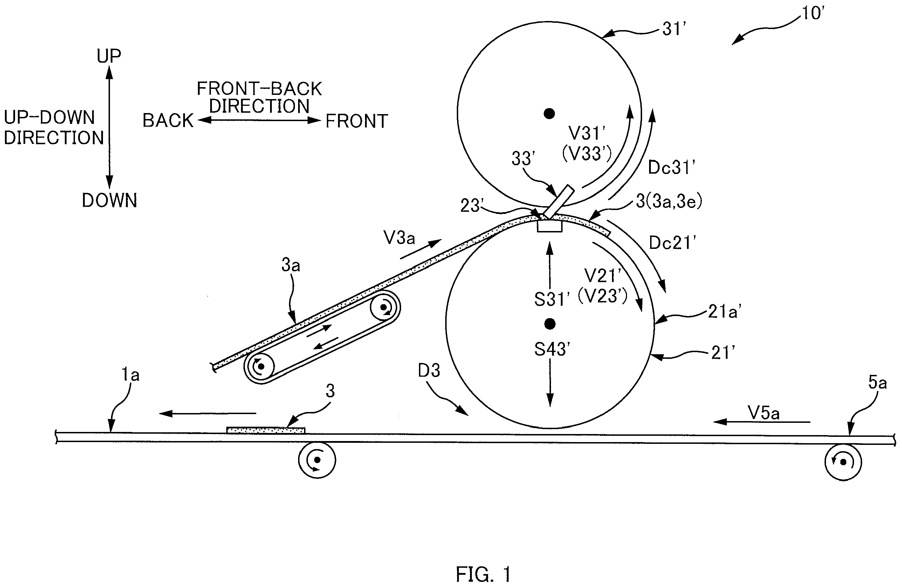

A slip cutting device 10' is one example of an apparatus for performing such processing. FIG 1 is a schematic side view of this apparatus 10'. This apparatus 10' has an anvil roll 21' that rotates in a rotation direction Dc21' that conforms to the conveying direction of a first continuous sheet 3a, and a cutter roll 31' that is arranged at a first predetermined position S31' in the rotation direction Dc21' of the anvil roll 21', and that rotates in a rotation direction Dc31' that conforms to the conveying direction.

Here, the anvil roll 21' rotates at a constant rotation speed value V21' (mpm). Also, multiple suction holes (not shown) that suction and hold the first continuous sheet 3a are provided in an outer circumferential surface 21a' of the anvil roll 21'. Furthermore, a first conveying speed value V3a of the first continuous sheet 3a being conveyed toward the outer circumferential surface 21a' is set to a speed value that is lower than the rotation speed value V21'. Accordingly, at the outer circumferential surface 21a' of the anvil roll 21', a leading end portion 3e of the first continuous sheet 3a is held in surface contact with the outer circumferential surface 21a' while also sliding thereon upstream in the rotation direction Dc21'.

Also, one cutter blade 33' is provided in an outer circumferential surface 31a' of the cutter roll 31' , and one receiving blade 23' having a surface for receiving the cutter blade 33' is provided in a corresponding location in the outer circumferential surface 21a' of the anvil roll 21' . When this receiving blade 23' passes the first predetermined position S31', which is the position where the cutter roll 31' is located in the rotation direction Dc21' of the anvil roll 21', the first continuous sheet 3a becomes clamped between the receiving blade 23' and the cutter blade 33' of the cutter roll 31', thus cutting the first continuous sheet 3a and cutting away the leading end portion 3e, thereby producing a cutform sheet 3.

Thereafter, the cutform sheet 3 cut away from the first continuous sheet 3a is subsequently held in a non-sliding manner on the outer circumferential surface 21a' of the anvil roll 21', and thus conveyed in the rotation direction Dc21' at the rotation speed value V21' of the anvil roll 21'. At this time, an interval D3 is formed between the aforementioned cutform sheet and the cutform sheet 3 that is cut and produced thereafter due to the difference between the rotation speed value V21' and the first conveying velocity value V3a.

Also, the second continuous sheet 5a is conveyed in a conveying direction that conforms to the rotation direction Dc21', at a second predetermined position S43' that is downstream of the first predetermined position S31' in the rotation direction Dc21' of the anvil roll 21' . Accordingly, when the cutform sheet 3 on the outer circumferential surface 21a' of the anvil roll 21' passes the second predetermined position S43' , the cutform sheet 3 is bonded and transferred to the second continuous sheet 5a.

Note that here, from the viewpoint of suppressing the formation of a wrinkle in the cutform sheet 3 or the second continuous sheet 5a during this transfer, the speed value (mpm) in the rotation direction Dc21' of the receiving blade 23' of the anvil roll 21' in the second predetermined position S43' needs to be matched with a second conveying speed value V5a of the second continuous sheet 5a, and this configuration is normally employed.

However, as previously mentioned, the rotation speed value V21' (mpm) of the anvil roll 121 is constant, and the rotation speed value V31' (mpm) of the cutter roll 31' is also constant. Accordingly, at the second predetermined position S43', the speed value V23' (mpm) in the rotation direction Dc21' of the receiving blade 23'is matched with the second conveying speed value V5a, and therefore at the first predetermined position S31' as well, at which the first continuous sheet 3a is to be cut, the receiving blade 23' of the anvil roll 21' and the cutter blade 33' of the cutter roll 31' respectively move in the rotation directions Dc21' and Dc31' at the same speed value as the second speed value V5a, which is higher than the first conveying velocity value V3a of the first continuous sheet 3a.

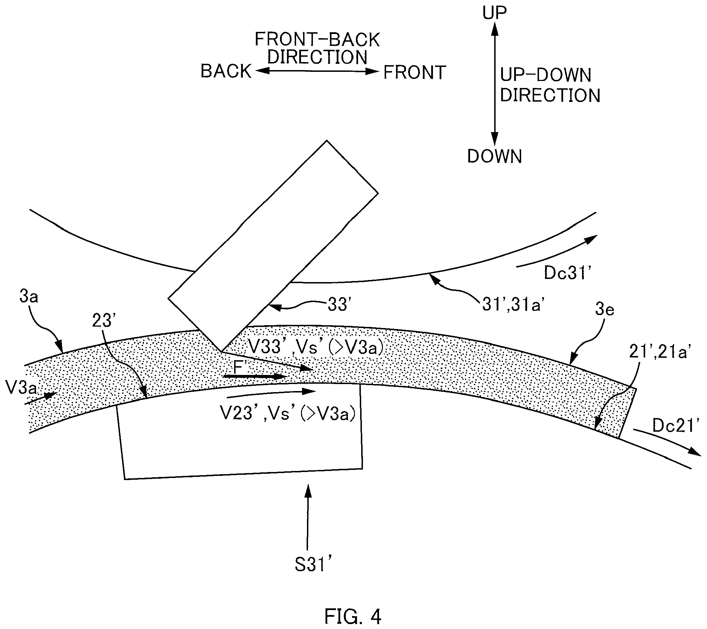

Accordingly, when the first continuous sheet 3a is cut with the receiving blade 23' and the cutter blade 33' , due to the difference between the first conveying velocity value V3a of the first continuous sheet 3a and the speed values V23' and V33' of the receiving blade 23' and the cutter blade 33', the first continuous sheet 3a is subjected to tensile force F' (e.g., see FIG. 4) that is directed downstream in the rotation directions Dc21' and Dc31' from the receiving blade 23' and the cutter blade 33' , and if the aforementioned difference becomes excessively large, it is possible for the tensile force F' to also become excessively large. As a result, there is a risk of instability in the cutting, such as the first continuous sheet 3a becoming ripped during cutting.

The present invention was achieved in light of conventional problems such as those described above, and an object of the present invention is to achieve improved stability in cutting by reducing the difference between the first conveying velocity value of the first continuous sheet and the speed values in the rotation directions of the receiving blade and the cutter blade when the first continuous sheet is cut.

### Solution to Problem

A primary aspect of the invention for achieving the aforementioned objective is a method for manufacturing a composite body of continuous sheets pertaining to an absorbent article, in which a first continuous sheet is cut to produce a cutform sheet, and the cutform sheet is bonded and transferred to a second continuous sheet while forming an interval on the second continuous sheet between the cutform sheet and another cutform sheet that is adjacent thereto in a continuity direction of the second continuous sheet, the method including:
conveying the first continuous sheet at a first conveying speed value (mpm) in a conveying direction that is a continuity direction of the first continuous sheet; rotating a holding roll with use of a motor in a rotation direction that conforms to the conveying direction of the first continuous sheet, the holding roll being rotated while the first continuous sheet is held on an outer circumferential surface of the holding roll such that the first continuous sheet slides upstream in the rotation direction on the outer circumferential surface;
rotating a cutter roll with use of a motor separate from the motor for the holding roll, the cutter roll being arranged in a first predetermined position in the rotation direction of the holding roll and being rotated in a rotation direction that conforms to the conveying direction;
cutting and producing the cutform sheet with a receiving blade in the outer circumferential surface of the holding roll cutting the first continuous sheet in conjunction with a cutter blade of the cutter roll when the receiving blade passes the first predetermined position in the rotation direction of the holding roll; and
transferring the cutform sheet on the outer circumferential surface to the second continuous sheet and bonding the cutform sheet on the second continuous sheet when the cutform sheet held in a non-sliding manner on the outer circumferential surface passes a second predetermined position in the rotation direction, the second continuous sheet being conveyed toward the second predetermined position at a second conveying speed value (mpm) that is higher than the first conveying speed value (mpm),
wherein the number of receiving blades in the rotation direction on the holding roll and the number of cutter blades in the rotation direction on the cutter roll are identical,
an arrangement pitch in the rotation direction of the receiving blade on a revolving path drawn with the receiving blade due to rotation of the holding roll in the rotation direction is identical to an arrangement pitch in the rotation direction of the cutter blade in a revolving path drawn with the cutter blade due to rotation of the cutter roll in the rotation direction, and
the holding roll rotates based on a defined speed pattern such that a speed value (mpm) in the rotation direction of the receiving blade when passing the second predetermined position is identical to the second conveying speed value, and such that a speed value (mpm) in the rotation direction of the receiving blade when passing the first predetermined position is lower than the speed value (mpm) in the rotation direction of the receiving blade when passing the second predetermined position, and in the cutting and producing, the speed value (mpm) in the rotation direction of the receiving blade when passing the first predetermined position is higher than the first conveying speed value of the first continuous sheet.

Further, an apparatus for manufacturing a composite body of continuous sheets pertaining to an absorbent article, in which a first continuous sheet is cut to produce a cutform sheet, and the cutform sheet is bonded and transferred to a second continuous sheet while forming an interval on the second continuous sheet between the cutform sheet and another cutform sheet that is adjacent thereto in a continuity direction of the second continuous sheet, the apparatus including:
a first conveying mechanism that conveys the first continuous sheet at a first conveying speed value (mpm) in a conveying direction that is a continuity direction of the first continuous sheet;
a holding roll mechanism having a holding roll that is driven to rotate with use of a motor in a rotation direction that conforms to the conveying direction of the first continuous sheet, the holding roll mechanism rotating the holding roll while the first continuous sheet is held on an outer circumferential surface of the holding roll such that the first continuous sheet slides upstream in the rotation direction on the outer circumferential surface; and
a cutter roll that is arranged in a first predetermined position in the rotation direction of the holding roll, the cutter roll being driven to rotate in a rotation direction that conforms to the conveying direction, with use of a motor separate from the motor for the holding roll,
wherein the cutform sheet is produced by a receiving blade at the outer circumferential surface of the holding roll cutting the first continuous sheet in conjunction with a cutter blade of the cutter roll, in the speed value (mpm) in the rotation direction higher than the first conveying speed value of the first continuous sheet, when the receiving blade passes the first predetermined position in the rotation direction of the holding roll,
the cutform sheet on the outer circumferential surface is bonded and transferred to the second continuous sheet when the cutform sheet held in a non-sliding manner on the outer circumferential surface passes a second predetermined position in the rotation direction, the second continuous sheet being conveyed toward the second predetermined position at a second conveying speed value (mpm) that is higher than the first conveying speed value (mpm),
the number of receiving blades in the rotation direction on the holding roll and the number of cutter blades in the rotation direction on the cutter roll are identical,
an arrangement pitch in the rotation direction of the receiving blade on a revolving path drawn with the receiving blade due to rotation of the holding roll in the rotation direction is identical to an arrangement pitch in the rotation direction of the cutter blade in a revolving path drawn with the cutter blade due to rotation of the cutter roll in the rotation direction, and
the holding roll rotates based on a defined speed pattern such that a speed value (mpm) in the rotation direction of the receiving blade when passing the second predetermined position is identical to the second conveying speed value, and such that a speed value (mpm) in the rotation direction of the receiving blade when passing the first predetermined position is lower than the speed value (mpm) in the rotation direction of the receiving blade when passing the second predetermined position.

Other features of the present invention will become apparent from the present description and the attached drawings.

### Advantageous Effects of Invention

According to the present invention, it is possible to achieve improved stability in cutting by reducing the difference between the first conveying velocity value of the first continuous sheet and the speed values in the rotation directions of the receiving blade and the cutter blade when the first continuous sheet is cut.

### Brief Description of the Drawings

FIG. 1 is a schematic side view of a conventional manufacturing apparatus 10'.
In FIG. 2, FIG. 2A is a schematic plan view of a back-face sheet 1, and FIG. 2B is a schematic plan view of an intermediate component 1a from which the back-face sheet 1 is obtained.
FIG. 3 is a schematic side view of a manufacturing apparatus 10 of an embodiment.
FIG. 4 illustrates a problem in a conventional manufacturing apparatus 10' and is a schematic side view showing an enlargement of the vicinity of a first predetermined position S31', which corresponds to a cut processing position S31.
FIG. 5A is a diagram illustrating a speed pattern for an anvil roll 21 and a cutter roll 31 according to an embodiment.
FIG. 5B is a schematic side view of the same manufacturing apparatus 10 as in FIG. 3, and is for assisting a description given using FIG. 5A.
FIG. 6A is a schematic side view of a manufacturing apparatus 10a of a first variation.
FIG. 6B is a diagram illustrating a speed pattern used in the apparatus 10a.
FIG. 7A is a schematic side view of a manufacturing apparatus 10b of a second variation.
FIG. 7B is a diagram illustrating a speed pattern used in the apparatus 10b.

### Description of Embodiments

At least the following matters are made clear from the present description and the attached drawings.

Disclosed is a method for manufacturing a composite body of continuous sheets pertaining to an absorbent article, in which a first continuous sheet is cut to produce a cutform sheet, and the cutform sheet is bonded and transferred to a second continuous sheet while forming an interval on the second continuous sheet between the cutform sheet and another cutform sheet that is adjacent thereto in a continuity direction of the second continuous sheet, the method comprising:
conveying the first continuous sheet at a first conveying speed value (mpm) in a conveying direction that is a continuity direction of the first continuous sheet;
rotating a holding roll with use of a motor in a rotation direction that conforms to the conveying direction of the first continuous sheet, the holding roll being rotated while the first continuous sheet is held on an outer circumferential surface of the holding roll such that the first continuous sheet slides upstream in the rotation direction on the outer circumferential surface;
rotating a cutter roll with use of a motor separate from the motor for the holding roll, the cutter roll being arranged in a first predetermined position in the rotation direction of the holding roll and being rotated in a rotation direction that conforms to the conveying direction;
cutting and producing the cutform sheet with a receiving blade in the outer circumferential surface of the holding roll cutting the first continuous sheet in conjunction with a cutter blade of the cutter roll when the receiving blade passes the first predetermined position in the rotation direction of the holding roll; and
transferring the cutform sheet on the outer circumferential surface to the second continuous sheet and bonding the cutform sheet on the second continuous sheet when the cutform sheet held in a non-sliding manner on the outer circumferential surface passes a second predetermined position in the rotation direction, the second continuous sheet being conveyed toward the second predetermined position at a second conveying speed value (mpm) that is higher than the first conveying speed value (mpm),
wherein the number of receiving blades in the rotation direction on the holding roll and the number of cutter blades in the rotation direction on the cutter roll are identical,
an arrangement pitch in the rotation direction of the receiving blade on a revolving path drawn with the receiving blade due to rotation of the holding roll in the rotation direction is identical to an arrangement pitch in the rotation direction of the cutter blade in a revolving path drawn with the cutter blade due to rotation of the cutter roll in the rotation direction, and
the holding roll rotates based on a defined speed pattern such that a speed value (mpm) in the rotation direction of the receiving blade when passing the second predetermined position is identical to the second conveying speed value, and such that a speed value (mpm) in the rotation direction of the receiving blade when passing the first predetermined position is lower than the speed value (mpm) in the rotation direction of the receiving blade when passing the second predetermined position.

According to this method for manufacturing a composite body of continuous sheets pertaining to an absorbent article, the speed value (mpm) in the rotation direction of the receiving blade when passing the first predetermined position is lower than the speed value (mpm) in the rotation direction of the receiving blade when passing the second predetermined position. For this reason, it is possible for the speed value of the receiving blade at the second predetermined position to be matched with the second conveying speed value necessary for smooth transfer of the cutform sheet to the second continuous sheet, while also setting the speed values of the receiving blade and the cutter blade for cutting at the first predetermined position closer to the first conveying speed value that is lower than the second conveying speed value. In other words, it is possible to reduce the difference between the first conveying speed value of the first continuous sheet and the speed values of the receiving blade and the cutter blade during cutting. Accordingly, it is possible to reduce the tensile force in the rotation direction that can be applied to the first continuous sheet with the receiving blade and the cutter blade during cutting, thereby making it possible to improve stability during cutting.

Also, the holding roll and the cutter roll have separate dedicated motors. Accordingly, it is possible to reduce the moment of inertia of the holding roll and the cutter roll that can act on the motors, thus making it possible for the holding roll and the cutter roll to each rotate with higher response. As a result, the holding roll and the cutter roll can each rotate smoothly in accordance with the speed pattern.

According to the method for manufacturing a composite body of continuous sheets pertaining to an absorbent article,
wherein preferably the second conveying speed value of the second continuous sheet is kept constant, and
in the transferring, the speed value (mpm) in the rotation direction of the receiving blade is kept constant from a start to an end of transfer of the cutform sheet to the second continuous sheet.

According to this method for manufacturing a composite body of continuous sheets pertaining to an absorbent article, in the transferring , the speed value in the rotation direction of the receiving blade is kept constant when the cutform sheet is transferred to the second continuous sheet, as described above. Accordingly, the cutform sheet, which is held in a non-sliding manner on the outer circumferential surface of the holding roll, can be stably transferred to the second continuous sheet.

According to the method for manufacturing a composite body of continuous sheets pertaining to an absorbent article, wherein in the cutting and producing, the speed value (mpm) in the rotation direction of the receiving blade when passing the first predetermined position is higher than the first conveying speed value of the first continuous sheet.

According to this method for manufacturing a composite body of continuous sheets pertaining to an absorbent article, even if the first continuous sheet is a material that has poor ease-of-cutting, the sheet can be cut reliably. This will be described in detail below. First, as a comparative example, if the speed value of the receiving blade is set to the same value as the first conveying speed value, the first continuous sheet is substantially not subjected to tensile force in the rotation direction from the receiving blade and the cutter blade. Accordingly, due to the fact that the first continuous sheet is a material that has poor ease-of-cutting, if a portion fails to be cut by clamping between the receiving blade and the cutter blade, the tensile force cannot compensate for the failure to cut that portion. As a result, the first continuous sheet may fail to be cut. However, in light of this, if the speed value of the receiving blade is set higher than the first conveying speed value as described above, the receiving blade and the cutter blade can apply an appropriate amount of tensile force to the first continuous sheet during cutting. Accordingly, even if a portion fails to be cut by clamping, that uncut portion can be cut by the tensile force. As a result, even if the first continuous sheet is a material that has poor ease-of-cutting, the sheet can be cut reliably.

According to the method for manufacturing a composite body of continuous sheets pertaining to an absorbent article, wherein preferably the speed value (mpm) in the rotation direction of the receiving blade when passing the first predetermined position is higher than an arithmetic mean of the first conveying speed value and the second conveying speed value.

According to this method for manufacturing a composite body of continuous sheets pertaining to an absorbent article, the speed value of the receiving blade when passing the first predetermined position is set closer to the second conveying speed value that is higher than the first conveying speed value. Accordingly, when the first continuous sheet is cut, the first continuous sheet can be reliably subjected to the tensile force in the rotation direction from the receiving blade and the cutter blade. As a result, even if the first continuous sheet is a material that has poor ease-of-cutting, the sheet can be cut reliably.

According to the method for manufacturing a composite body of continuous sheets pertaining to an absorbent article, wherein preferably the speed value (mpm) in the rotation direction of the receiving blade when passing the first predetermined position is lower than an arithmetic mean of the first conveying speed value and the second conveying speed value.

According to this method for manufacturing a composite body of continuous sheets pertaining to an absorbent article, the speed value of the receiving blade when passing the first predetermined position is set closer to the first conveying speed value that is lower than the second conveying speed value. Accordingly, it is possible to reliably reduce the difference between the first conveying speed value of the first continuous sheet and the speed values of the receiving blade and the cutter blade when the first continuous sheet is cut. Accordingly, it is possible to reliably reduce the tensile force in the rotation direction that can be applied to the first continuous sheet by the receiving blade and the cutter blade during cutting, thereby making it possible to reliably improve stability during cutting.

According to the method for manufacturing a composite body of continuous sheets pertaining to an absorbent article,
wherein preferably the cutter blade protrudes from an outer circumferential surface of the cutter roll, and
an adhesive for bonding of the second continuous sheet and the cutform sheet is applied to, out of two surfaces of the first continuous sheet, a surface that faces the cutter blade.

According to this method for manufacturing a composite body of continuous sheets pertaining to an absorbent article, in the cutting and producing, the speed value (mpm) of the receiving blade when passing the first predetermined position is higher than the first conveying speed value of the first continuous sheet. For this reason, compared with the case where the speed value of the receiving blade is set to the same value as the first conveying speed value, it is possible to reduce the time for which the cutter blade is in contact with adhesive on the first continuous sheet during the cutting of the first continuous sheet, thus making it possible to suppress the attachment of the adhesive to the cutter blade. As a result, it is possible to prevent the adhesive from becoming deposited on the cutter blade and reducing the cutting performance of the cutter blade. It is also possible to prevent the case where the adhesive deposited on the cutter blade attaches to the cut and produced cutform sheet and soils it.

According to the method for manufacturing a composite body of continuous sheets pertaining to an absorbent article, wherein preferably in the cutting and producing, the speed value (mpm) in the rotation direction of the receiving blade is kept constant from a start to an end of cutting of the first continuous sheet with the cutter blade and the receiving blade.

According to this method for manufacturing a composite body of continuous sheets pertaining to an absorbent article, the speed value (mpm) in the rotation direction of the receiving blade is kept constant from the start to the end of the cutting of the first continuous sheet. Accordingly, the first continuous sheet can be cut stably.

According to the method for manufacturing a composite body of continuous sheets pertaining to an absorbent article, wherein preferably the first conveying speed value of the first continuous sheet is kept constant.

According to this method for manufacturing a composite body of continuous sheets pertaining to an absorbent article, the first conveying speed value of the first continuous sheet is kept constant. Accordingly, it is possible to suppress an oscillating increase in the tensile force applied to the first continuous sheet that can occur in the case where the first conveying speed value fluctuates, thus making it possible to improve stability in cutting. It is also possible to suppress damage to the first continuous sheet that can occur in the case where the first conveying speed value fluctuates.

According to the method for manufacturing a composite body of continuous sheets pertaining to an absorbent article,
wherein preferably the receiving blade is a member separate from the anvil roll,
the receiving blade has an arc surface that extends along the rotation direction of the holding roll, and
the first continuous sheet is clamped between and cut with the arc surface and the cutter blade.

According to this method for manufacturing a composite body of continuous sheets pertaining to an absorbent article, the receiving blade has an arc surface that extends along the rotation direction of the holding roll, and the first continuous sheet is cut using the arc surface. Accordingly, there are cases where a synchronization problem arises in the rotation of the holding roll and the cutter roll due to the fact that the holding roll and the cutter roll are driven to rotate by separate motors, and the receiving blade and the cutter blade become shifted in the rotation direction from the relative positions that they are originally to be located at, and even in such a case, the receiving blade can receive the cutter blade with the arc surface for cutting, without a big problem occurring. For this reason, even in such a case, the first continuous sheet can be cut without a big problem occurring.

According to the method for manufacturing a composite body of continuous sheets pertaining to an absorbent article,
wherein preferably a plurality of receiving blades and a plurality of cutter blades are provided, and
the second predetermined position is set in a position in the rotation direction of the holding roll that is a position in which, when one of the plurality of receiving blades passes the first predetermined position, another one of the receiving blades is not located.

According to this method for manufacturing a composite body of continuous sheets pertaining to an absorbent article, the speed value of the receiving blade when passing the first predetermined position is set different from the speed value of the receiving blade when passing the second predetermined position. Accordingly, the speed pattern of the holding roll can be easily set such that the speed value of the receiving blade when passing the first predetermined position is lower than the speed value of the receiving blade when passing the second predetermined position.

Disclosed is an apparatus for manufacturing a composite body of continuous sheets pertaining to an absorbent article, in which a first continuous sheet is cut to produce a cutform sheet, and the cutform sheet is bonded and transferred to a second continuous sheet while forming an interval on the second continuous sheet between the cutform sheet and another cutform sheet that is adjacent thereto in a continuity direction of the second continuous sheet, the apparatus comprising:
a first conveying mechanism that conveys the first continuous sheet at a first conveying speed value (mpm) in a conveying direction that is a continuity direction of the first continuous sheet;
a holding roll mechanism having a holding roll that is driven to rotate with use of a motor in a rotation direction that conforms to the conveying direction of the first continuous sheet, the holding roll mechanism rotating the holding roll while the first continuous sheet is held on an outer circumferential surface of the holding roll such that the first continuous sheet slides upstream in the rotation direction on the outer circumferential surface; and
a cutter roll that is arranged in a first predetermined position in the rotation direction of the holding roll, the cutter roll being driven to rotate in a rotation direction that conforms to the conveying direction, with use of a motor separate from the motor for the holding roll,
wherein the cutform sheet is produced by a receiving blade at the outer circumferential surface of the holding roll cutting the first continuous sheet in conjunction with a cutter blade of the cutter roll when the receiving blade passes the first predetermined position in the rotation direction of the holding roll,
the cutform sheet on the outer circumferential surface is bonded and transferred to the second continuous sheet when the cutform sheet held in a non-sliding manner on the outer circumferential surface passes a second predetermined position in the rotation direction, the second continuous sheet being conveyed toward the second predetermined position at a second conveying speed value (mpm) that is higher than the first conveying speed value (mpm),
the number of receiving blades in the rotation direction on the holding roll and the number of cutter blades in the rotation direction on the cutter roll are identical,
an arrangement pitch in the rotation direction of the receiving blade on a revolving path drawn with the receiving blade due to rotation of the holding roll in the rotation direction is identical to an arrangement pitch in the rotation direction of the cutter blade in a revolving path drawn with the cutter blade due to rotation of the cutter roll in the rotation direction, and
the holding roll rotates based on a defined speed pattern such that a speed value (mpm) in the rotation direction of the receiving blade when passing the second predetermined position is identical to the second conveying speed value, and such that a speed value (mpm) in the rotation direction of the receiving blade when passing the first predetermined position is lower than the speed value (mpm) in the rotation direction of the receiving blade when passing the second predetermined position.

According to this apparatus for manufacturing a composite body of continuous sheets pertaining to an absorbent article, it is possible to achieve actions and effects similar to the case of the manufacturing method described above.

### Embodiments

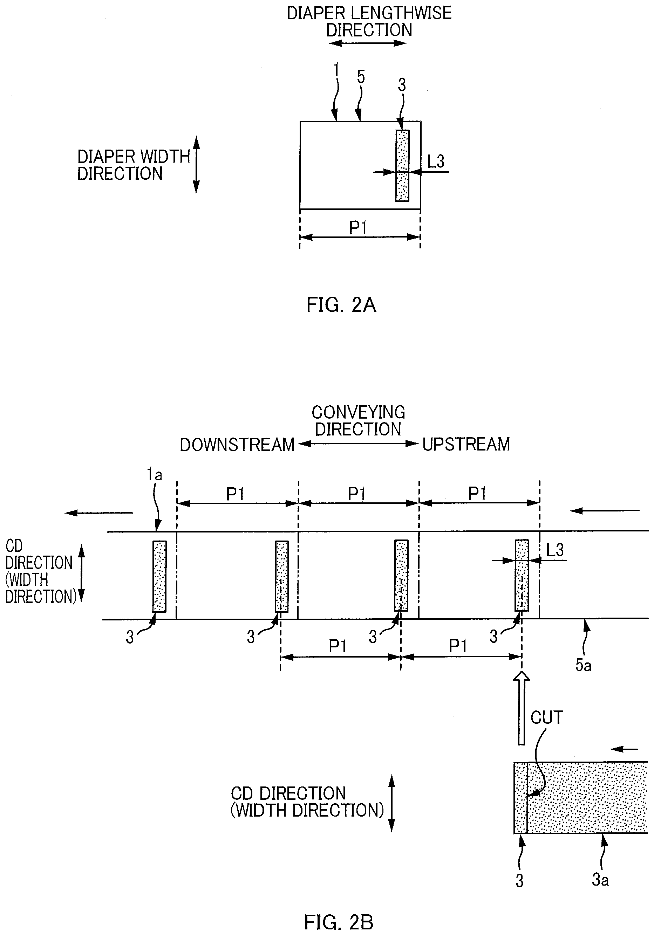

A manufacturing method and a manufacturing apparatus 10 of the present embodiment, which are for manufacturing a composite body of continuous sheets pertaining to an absorbent article, are applied to the manufacturing of an intermediate component 1a from which a back-face sheet 1 of a disposable diaper is obtained, as one example of the composite body of continuous sheets. FIG. 2A is a schematic plan view of the back-face sheet 1, and FIG. 2B is a schematic plan view of the intermediate component 1a from which the back-face sheet 1 is obtained.

This disposable diaper is a so-called tape-type diaper. Specifically, this is a type of diaper in which a pair of pieces of fastening tape (not shown) located on the two width-direction sides of the diaper are fastened to a piece of target tape when this diaper is put on the wearer.

For this reason, this back-face sheet 1 has an exterior sheet 5 that forms the exterior of the diaper, and a piece of target tape 3 that is bonded to a surface of the exterior sheet 5 on the outer side in the thickness direction (the surface located on the non-skin side with respect to the wearer) . Also, a liquid-impermeable leak-proof sheet (not shown), an absorbent body (not shown) formed by pulp fibers, a liquid-permeable top face sheet (not shown), and the like are successively fixed in an overlaid manner on the surface of the exterior sheet 5 on the inner side in the thickness direction (the surface located on the skin side with respect to the wearer), thus producing the base part of the diaper.

Note that the material forming the exterior sheet 5 can be a soft nonwoven fabric that is mainly constituted by resin fibers, for example, and a spunbonded nonwoven fabric is used here. Also, the material forming the target tape 3 can be a material that can appropriately engage with the male components (hook members) of a fastening tape, and examples include the female components (loop members) of a hook-and-loop fastener and an air-through nonwoven fabric. An air-through nonwoven fabric is used here.

As shown in FIG. 2B, the intermediate component 1a from which the back-face sheet 1 is obtained is a continuous body before being cut at a product pitch P1 to form individual back-face sheets 1. Specifically, the intermediate component 1a includes a continuous web 5a that is the original fabric for the exterior sheet 5, and multiple rectangular pieces of target tape 3 that are bonded thereon side-by-side at the product pitch P1 in the continuity direction of the continuous web 5a. This intermediate component 1a is manufactured with the manufacturing method and the manufacturing apparatus 10 of the present embodiment.

Specifically, according to the manufacturing method and the manufacturing apparatus 10, first, a target tape continuous body 3a, which is the original fabric for the target tape 3, is cut to produce rectangular pieces of target tape 3, and these pieces of target tape 3 are bonded onto the continuous web 5a, which is the original fabric for the exterior sheet 5, at the product pitch P1 in the continuity direction thereof, thus manufacturing the intermediate component 1a.

The manufacturing apparatus 10 will be described in detail below.

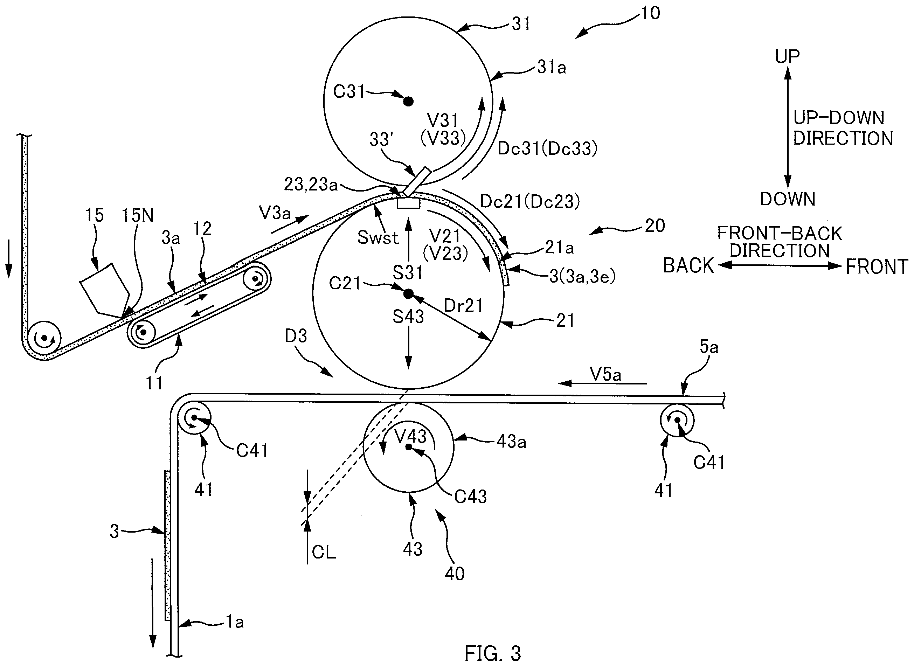

FIG. 3 is a schematic side view of the manufacturing apparatus 10 of the present embodiment. In this manufacturing apparatus 10, a CD direction (in FIG. 3, the direction passing through the paper plane) is set as the width direction of the apparatus 10. Also, in this example, the CD direction is oriented in the horizontal direction, but there is no limitation whatsoever to this. Also, in this example, a vertical up-down direction and a horizontal front-rear direction are set as the two directions that are orthogonal to the CD direction, and therefore the conveying directions of the target tape continuous body 3a and the exterior sheet continuous web 5a are oriented in directions defined by both the up-down direction and the front-rear direction. Also, the width directions of the target tape continuous body 3a and the exterior sheet continuous web 5a are each parallel with the CD direction. Furthermore, letting the direction orthogonal to the CD direction and the conveying direction be defined as a Z direction, this Z direction is parallel with the thickness directions of the target tape continuous body 3a and the exterior sheet continuous web 5a.

Also, in this example, the "target tape continuous body 3a" and the "target tape 3" respectively correspond to the "first continuous sheet" and the "cutform sheet" in the claims, and the "continuous web 5a" corresponds to the "second continuous sheet" in the claims.

As shown in FIG. 3, this manufacturing apparatus 10 is a slip cutting device. Specifically, this apparatus 10 has (1) a target tape conveying mechanism 11 that conveys the target tape continuous body 3a along the conveying direction at a first conveying speed value V3a, (2) an adhesive application device 15 that applies an adhesive (not shown) to one of the two surfaces of the target tape continuous body 3a, (2) an anvil roll mechanism 20 that, by rotating an anvil roll 21 (corresponding to a holding roll) at a rotation speed value V21 that is higher than the first conveying velocity value V3a, holds the target tape continuous body 3a, which is conveyed from the target tape conveying mechanism 11, on an outer circumferential surface 21a of the anvil roll 21 while causing the target tape continuous body 3a to slide upstream in a rotation direction Dc21 on the outer circumferential surface 21a, (3) a cutter roll 31 that produces rectangular pieces of target tape 3 from the target tape continuous body 3a by clamping and cutting the target tape continuous body 3a along with a receiving blade 23 provided in the outer circumferential surface 21a of the anvil roll 21, and (4) a continuous web conveying mechanism 40 that, when the target tape 3 has been cut from the target tape continuous body 3a and is being held in a non-sliding manner on the outer circumferential surface 21a of the anvil roll 21, conveys a continuous web 5a of the exterior sheet 5 toward the target tape 3 at a second conveying speed value V5a (mpm) that is higher than the first conveying speed value V3a (mpm) in a conveying direction that conforms to the rotation direction Dc21 of the anvil roll 21, such that the target tape 3 is bonded and transferred to the continuous web 5a with use of the adhesive.

The target tape conveying mechanism 11 (corresponds to a first conveying mechanism) is a suction belt conveyer, for example. Specifically, it has an endless belt 12 with suction holes (not shown) formed in the outer circumferential surface, which is the conveying surface, and the target tape continuous body 3a is suctioned to be in surface contact with the outer circumferential surface of the endless belt 12 by suction through the suction holes. In this suctioned state, the endless belt 12 is driven to rotate with use of a servomotor as the drive source, thus conveying the target tape continuous body 3a in the conveying direction at the first conveying velocity value V3a (mpm) that is maintained at a constant value.

It should be noted that the target tape conveying mechanism 11 is not limited in any way to being a suction belt conveyer. For example, a pinch roll apparatus (not shown) may be used. Specifically, with this pinch roll apparatus, the target tape continuous body 3a is clamped and conveyed with a pair of upper and lower driving rollers that rotate in opposite directions.

The adhesive application device 15 has a nozzle 15N that ejects an adhesive such as a hot-melt adhesive, and a pump (not shown) that feeds the adhesive to the nozzle 15N. Due to the adhesive being ejected from the nozzle 15N, the adhesive is applied to one of the two surfaces of the target tape continuous body 3a.

Examples of the adhesive application pattern include an Ω pattern in which wavy lines extending in the conveying direction are arranged side-by-side in the CD direction, a stripe pattern in which straight lines extending in the conveying direction are arranged side-by-side in the CD direction, and a spiral pattern in which spiral lines extending in the conveying direction are arranged side-by-side in the CD direction, and the Ω pattern is used here. Also, in this example, the adhesive is applied over approximately the entire range in the CD direction so as to reach both ends in the CD direction, but there is no limitation whatsoever to this.

The anvil roll mechanism 20 (corresponding to the holding roll mechanism) has the previously described anvil roll 21. This roll 21 is supported to be capable of rotating about a rotation axis C21 that conforms to the CD direction, and therefore the roll 21 can rotate in a rotation direction Dc21 that conforms to the conveying direction of the target tape continuous body 3a. Also, a servomotor (not shown) is coupled to the roll 21, and the roll 21 is driven to rotate in the rotation direction Dc21 when drive rotation force is received from the motor.

Here, the outer circumferential surface 21a of the anvil roll 21 has a holding function for wrapping and holding a sheet-shaped object in surface contact, and therefore holds the target tape continuous body 3a and the rectangular target tape 3 in surface contact. In this example, this holding function is realized by suction holes (not shown) formed in the outer circumferential surface 21a. Specifically, due to suction through these suction holes, the outer circumferential surface 21a of the anvil roll 21 produces suction force, and this suction force becomes holding force for holding the target tape continuous body 3a and the rectangular target tape 3. It should be noted that the method for giving holding force to the outer circumferential surface 21a is not limited whatsoever to this, and another method that employs attraction by static electricity or the like may be used.

The above-described target tape continuous body 3a is conveyed on the outer circumferential surface 21a of the anvil roll 21 in a direction approximately tangential to the outer circumferential surface 21a, and the continuous body 3a is wrapped and held in surface contact on the outer circumferential surface 21a by the above-described holding force. Note that a wrapping start position Swst of the continuous body 3a on the outer circumferential surface 21a is located in a position that is a predetermined angle upstream from an arrangement position S31 of the cutter roll 31 in the rotation direction Dc21.

Here, the rotation speed value V21 (mpm) of the anvil roll 21 is set to a speed value that is higher than the first conveying speed value V3a of the target tape continuous body 3a as previously described. Accordingly, the target tape continuous body 3a is held in surface contact on the outer circumferential surface 21a of the anvil roll 21 so as to slide upstream on the outer circumferential surface 21a. In other words, while sliding over the outer circumferential surface 21a, the target tape continuous body 3a moves gradually downstream in the rotation direction Dc21 based on the above-described first conveying speed value V3a.

Meanwhile, the outer circumferential surface 21a of the anvil roll 21 is provided with only one receiving blade 23 that receives a later-described cutter blade 33 of the cutter roll 31. Also, in this example, the receiving blade 23 has an arc surface 23a that faces outward in a radius of rotation direction Dr21 of the anvil roll 21, and furthermore the arc surface 23a is in a concentric relationship with the outer circumferential surface 21a of the anvil roll 21 and has an arc shape with the same radius as the outer circumferential surface 21a, and therefore the arc surface 23a and the outer circumferential surface 21a are in a so-called flush relationship. When this receiving blade 23 passes the arrangement position S31 (corresponding to a first predetermined position) of the cutter roll 31 in the rotation direction Dc21, the arc surface 23a of the receiving blade 23 clamps the target tape continuous body 3a on the outer circumferential surface 21a along with the cutter blade 33 of the cutter roll 31, which rotates in coordination with the anvil roll 21, thus cutting the target tape continuous body 3a and cutting away a leading end portion 3e thereof. The cut-away leading end portion 3e is the rectangular piece of target tape 3.

Note that this cut-away piece of target tape 3 is subsequently held in a non-sliding manner on the receiving blade 23 and the outer circumferential surface 21a of the anvil roll 21, thus being conveyed downstream in the rotation direction Dc21 at the rotation speed value V21 of the anvil roll 21 along with the outer circumferential surface 21a and the receiving blade 23. At this time, an interval D3 is formed between this piece of target tape 3 and the subsequently cut away piece of target tape 3, based on the difference between the rotation speed value V21 and the first conveying speed value V3a of the target tape continuous body 3a.

It should be noted that in this example, as shown in FIG. 3, the receiving blade 23 is configured as a member that is separate from the anvil roll 21 and is removable from the roll 21. Accordingly, when the receiving blade 23 becomes worn down, it is sufficient to replace merely the receiving blade 23, or in other words, there is no need to replace the anvil roll 21 itself. This therefore achieves a reduction in maintenance cost.

The cutter roll 31 is arranged in the predetermined position S31 in the rotation direction Dc21 of the anvil roll 21, as previously mentioned. This roll 31 is supported to be capable of rotating about a rotation axis C31 that conforms to the CD direction, and therefore the roll 31 can rotate in a rotation direction Dc31 that conforms to the conveying direction of the target tape continuous body 3a. Also, the roll 31 is coupled to a servomotor (not shown) that is different from the servomotor of the anvil roll 21, and therefore the roll 31 is driven to rotate in the rotation direction Dc31 when receiving drive rotation force from the motor. In other words, the anvil roll 21 and the cutter roll 31 have separate dedicated servomotors. Accordingly, compared to the case where the two rolls 21 and 31 are driven by one servomotor, it is possible to reduce the moment of inertia of the anvil roll 21 and the cutter roll 31 that can act on the motors, thus making it possible for the anvil roll 21 and the cutter roll 31 to each rotate with higher response. As a result, the anvil roll 21 and the cutter roll 31 can each rotate smoothly in accordance with a later-described speed pattern.

Also, the outer circumferential surface 31a of the cutter roll 31 is provided with only one blade-shaped cutter blade 33 that extends in the CD direction, that is to say one cutter blade in correspondence with the one receiving blade 23 of the anvil roll 21. Furthermore, the arrangement pitch (m) of the receiving blade 23 in the rotation direction Dc21 on the revolving path drawn by the arc surface 23a of the receiving blade 23 due to rotation of the anvil roll 21 in the rotation direction Dc21 is deemed to be the same as the arrangement pitch (m) of the cutter blade 33 in the rotation direction Dc31 on the revolving path drawn with the blade tip of the cutter blade 33 due to rotation of the cutter roll 31 in the rotation direction Dc31. For example, in this example, one cutter blade 33 and one receiving blade 23 are provided, and therefore the arrangement pitch (m) for each of them is the circumferential length (m) of one revolution around the revolving path. Also, these circumferential lengths are the same value as each other.

Accordingly, based on the later-described speed pattern, the cutter roll 31 and the anvil roll 21 rotate such that the speed value V33 (mpm) in the rotation direction Dc31 of the cutter blade 33 matches the speed value V23 (mpm) in the rotation direction Dc21 of the receiving blade 23, and therefore the cutter roll 31 and the anvil roll 21 can clamp and cut the target tape continuous body 3a so as to produce a piece of target tape 3. Specifically, the cutter roll 31 can rotate such that the cutter blade 33 faces the receiving blade 23 when the receiving blade 23 of the anvil roll 21 passes the arrangement position S31 of the cutter roll 31 in the rotation direction Dc21, and therefore the receiving blade 23 and the cutter blade 33 can together clamp the target tape continuous body 3a. Note that hereinafter, the arrangement position S31 of the cutter roll 31 at which such cutting by clamping is performed will also be referred to as the "cut processing position S31".

The continuous web conveying mechanism 40 conveys the exterior sheet continuous web 5a along a predetermined conveying route. The continuous web 5a is conveyed toward a predetermined position S43 (corresponding to a second predetermined position) that is downstream of the arrangement position S31 of the cutter roll 31 in the rotation direction Dc21 of the anvil roll 21. Specifically, the continuous web 5a is conveyed along the conveying route such as to be closest to the outer circumferential surface 21a of the anvil roll 21 at the predetermined position S43. Accordingly, when the target tape 3, which is held in a non-sliding manner on the outer circumferential surface 21a of the anvil roll 21, passes the predetermined position S43, the target tape 3 is bonded and transferred to the exterior sheet continuous web 5a with use of the previously mentioned adhesive. Subsequently, the target tape 3 is conveyed in the conveying direction of the continuous web 5a together with the continuous web 5a, thus producing the intermediate component 1a of the back-face sheet 1. Note that hereinafter, the predetermined position S43 at which this transfer is performed will also be called the "transfer processing position S43".

In order to form this conveying route, the continuous web conveying mechanism 40 has three rolls 41, 43, and 41 that are side-by-side in the conveying direction of the continuous web 5a, for example. These rolls 41, 43, and 41 are supported to be capable of respectively rotating about rotation axes C41, C43, and C41 that conform to the CD direction. Also, the rolls 41, 43, and 41 are each driven to rotate when drive rotation force is received from a servomotor (not shown) . When the rolls 41, 43, and 41 come into contact with the continuous web 5a, the continuous web 5a is conveyed in the conveying direction by conveying force in the conveying direction received from the rolls 41, 43, and 41.

Here, among these three rolls 41, 43, and 41, the roll 43 located at the center in the conveying direction is arranged at a position in the vicinity of the transfer processing position S43. Also, a clearance CL between an outer circumferential surface 43a of this roll 43 and the outer circumferential surface 21a of the anvil roll 21 is set to a dimension according to which, when the target tape 3 held on the outer circumferential surface 21a of the anvil roll 21 passes the transfer processing position S43, the target tape 3 and the continuous web 5a are both somewhat clamped in the thickness direction with the anvil roll 21 and the roll 43.

Accordingly, when the target tape 3 held on the outer circumferential surface 21a of the anvil roll 21 passes the transfer processing position S43, the target tape 3 on the outer circumferential surface 21a and the continuous web 5a are clamped and bonded using the previously mentioned adhesive, and thus the target tape 3 is transferred from the outer circumferential surface 21a to the continuous web 5a.

Also, as previously mentioned, the second conveying speed value V5a of the continuous web 5a is set to a constant value that is higher than the previously mentioned first conveying speed value V3a of the target tape 3. For this reason, at the transfer processing position S43, the target tape 3 on the outer circumferential surface 21a of the anvil roll 21 can be bonded to the continuous web 5a such that the interval D3 is formed between it and the piece of target tape 3 that is adjacent on the downstream side in the conveying direction on the continuous web 5a.

It should be noted that, from the viewpoint of suppressing the formation of a wrinkle when the target tape 3 is bonded and transferred to the continuous web 5a, the rotation speed value V21 (mpm) of the anvil roll 21 needs to match the second conveying speed value V5a of the continuous web 5a when the target tape 3 passes the transfer processing position S43. In other words, the speed value V23 (mpm) in the rotation direction Dc21 of the receiving blade 23 needs to match the second conveying speed value V5a of the continuous web 5a at least when the receiving blade 23 passes the transfer processing position S43.

However, with the conventional slip cutting device 10' in FIG. 1, ordinarily the rotation speed value V21' (mpm) of the anvil roll 21' is maintained at a constant value over the entirety of the rotation direction Dc21' , and accordingly, the rotation speed value V31' (mpm) of the cutter roll 31' is also ordinarily maintained at a constant value over the entirety of the rotation direction Dc31' . For this reason, if the speed value V23' (mpm) of the receiving blade 23' is matched with the second conveying speed value V5a (mpm) at the previously mentioned second predetermined position S43' that corresponds to the transfer processing position S43, then due to this, as shown in the schematic side view in FIG. 4, at the previously mentioned first predetermined position S31' as well, which corresponds to the cut processing position S31 at which the target tape continuous body 3a is to be cut, the receiving blade 23' and the cutter blade 33' respectively move in the rotation directions Dc21' and Dc31' at the same speed value Vs' as the second conveying speed value V5a. However, this speed value Vs' is higher than the first conveying speed value V3a of the target tape continuous body 3a in order for cutting to be performed in the first predetermined position S31'.

For this reason, when the continuous body 3a is cut with the receiving blade 23' and the cutter blade 33' , due to the difference between the first conveying speed value V3a of the target tape continuous body 3a and the speed values V23' and V33' in the rotation directions Dc21' and Dc31' of the blades 23' and 33', the target tape continuous body 3a is subjected to tensile force F', which is directed downstream in the rotation directions Dc21' and Dc31' , from the blades 23' and 33'. However, if this difference become excessively high, the tensile force F' also becomes excessive, and as a result, there is a risk of cutting instability, such as the target tape continuous body 3a becoming ripped when being cut.

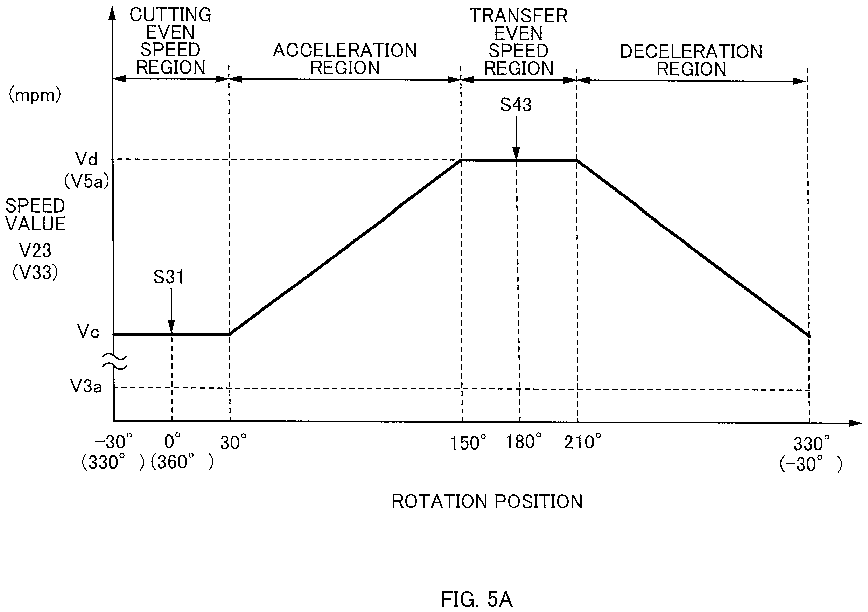

In view of this, in the present embodiment shown in FIG. 3, the anvil roll 21 is rotated based on a defined speed pattern (FIG. 5A), and therefore the speed value V23 in the rotation direction Dc21 of the receiving blade 23 is changed according to the position in the rotation direction Dc21 as described below. Specifically, the speed value V23 (mpm) in the rotation direction Dc21 of the receiving blade 23 when passing the transfer processing position S43 in FIG. 3 is set to the same value as the second conveying speed value V5a of the continuous web 5a, and the speed value V23 (mpm) in the rotation direction Dc21 of the receiving blade 23 when passing the cut processing position S31 is set lower than the speed value V23 (mpm) in the rotation direction Dc21 of the receiving blade 23 when passing the transfer processing position S43. Moreover, the cutter roll 31 is also rotated in coordination with the anvil roll 21 that rotates according to the aforementioned speed pattern. In other words, the cutter roll 31 is also rotated in coordination such that the speed value V33 in the rotation direction Dc31 of the cutter blade 33 is set to the same value as the speed value V23 in the rotation direction Dc21 of the receiving blade 23.

Accordingly, it is possible for the speed value V23 of the receiving blade 23 in the transfer processing position S43 to be matched with the second conveying speed value V5a necessary for smooth transfer of the target tape 3 to the continuous web 5a, while also setting the speed values V23 and V33 of the receiving blade 23 and the cutter blade 33 for cutting in the cut processing position S31 closer to the first conveying speed value V3a that is lower than the second conveying speed value V5a. In other words, it is possible to reduce the difference between the first conveying speed value V3a of the target tape continuous body 3a and the speed values V23 and V33 of the receiving blade 23 and the cutter blade 33 during cutting. Accordingly, it is possible to reduce the tensile force F' (FIG. 4) in the rotation directions Dc21 and Dc31 that can be applied to the target tape continuous body 3a with the receiving blade 23 and the cutter blade 33 during cutting, thereby making it possible to improve stability during cutting.

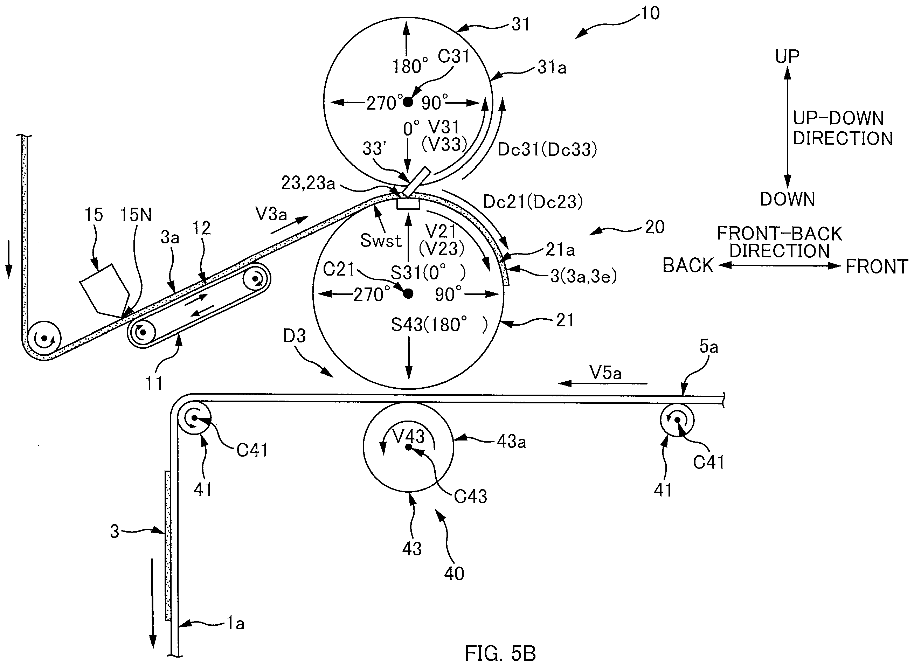

FIG. 5A is a diagram illustrating the aforementioned speed pattern for the anvil roll 21. Also, FIG. 5B is a diagram for assisting the description, and is a schematic side view of the same manufacturing apparatus 10 as in FIG. 3.

Note that the horizontal axis in FIG. 5A indicates the position of the receiving blade 23 (specifically, the central portion in the circumferential direction of the blade 23) in the rotation direction Dc21 of the anvil roll 21, and the vertical axis indicates the speed value V23 (mpm) in the rotation direction Dc21 of the receiving blade 23 (specifically, the arc surface 23a of the blade 23). Also, the position in the rotation direction Dc21 of the former (hereinafter, also called the rotation position) is indicated by a rotation angle (FIG. 5B) about the rotation axis C21 of the anvil roll 21. Moreover, as shown in FIG. 5B, letting the upper end position of the anvil roll 21 that is closest to the cutter roll 31 be "0°", and given that the anvil roll 21 rotates in the clockwise direction about the rotation axis C21, the rotation angle is divided into angles from 0° to 360° (0°) for rotation positions over one rotation in the clockwise direction, beginning with the upper end position 0°.

Moreover, the cutter roll 31 also rotates based on the aforementioned speed pattern. Accordingly, the horizontal axis in FIG. 5A indicates the position of the cutter blade 33 (specifically, the blade tip of the blade 33) in the rotation direction Dc31 of the cutter roll 31, and the vertical axis indicates the speed value V33 (mpm) in the rotation direction Dc31 of the cutter blade 33 (specifically, the blade tip of the blade 33). It should be noted that, as shown in FIG. 5B, the cutter roll 31 rotates in the counter-clockwise direction, which is opposite to the anvil roll 21, and is located directly above the anvil roll 21, and therefore the lower end position of the cutter roll 31 that is closest to the anvil roll 21 is considered to be the rotation position "0°", and the rotation angle is divided into angles from 0° to 360° (0°) for rotation positions over one rotation in the counter-clockwise direction, beginning at the lower end position.

This speed pattern is set such that one rotation of the anvil roll 21 and the cutter roll 31 is one cycle. Accordingly, the speed values V23 and V33 (mpm) in the rotation directions Dc21 and Dc31 of the receiving blade 23 and the cutter blade 33 are set for each of the rotation positions from 0° to 360°. The rolls 21 and 31 each repeatedly rotate one time in accordance with the above-described speed pattern.

Also, as previously described with reference to FIG. 5B, at the 0° rotation position, the anvil roll 21 and the cutter roll 31 are closest to each other, and the 0° rotation positions of the anvil roll 21 and the cutter roll 31 are the previously mentioned cut processing position S31. Also, in the case of the anvil roll 21, the previously mentioned wrapping start position Swst is located at a rotation position that is -20° upstream of the cut processing position S31 in the rotation direction Dc21 for example (i.e., 340°) . Furthermore, in the case of the same anvil roll 21, the previously mentioned transfer processing position S43 is located at the 180° rotation position that is in point symmetry with the cut processing position S31 about the rotation axis C21.

As shown in FIG. 5A, in this example, the speed pattern is a trapezoidal pattern. Specifically, this speed pattern has an even speed region that has a constant speed value, and corresponds to the 0° cut processing position S31 and includes this position S31 (hereinafter, also called the cutting even speed region) . The speed pattern also has an even speed region that has a constant speed value, and corresponds to the 180° transfer processing position S43 and includes this position S43 (hereinafter, also called the transfer even speed region) . Furthermore, at a position between the cutting even speed region and the transfer even speed region in the rotation direction Dc21, the speed pattern has an acceleration region in which the speed increases at a constant rate according to the rotation angle, and at a position between the transfer even speed region and the cutting even speed region in the rotation direction Dc21, the speed pattern has a deceleration region in which the speed decreases at a constant rate according to the rotation angle.

Also, the speed value Vc in the cutting even speed region is set to a speed value that is between the first conveying speed value V3a and the second conveying speed value V5a, and the speed value Vd in the transfer even speed region is set to the same speed value as the second conveying speed value V5a.

Accordingly, it is possible to achieve the previously described main actions and effects of the present embodiment. Specifically, it is possible to enable smooth transfer of the target tape to the continuous web 5a in the transfer processing position S43, while also improving stability in cutting in the cut processing position S31.

Furthermore, in this example, the speed value Vc in the cutting even speed region is set to a speed value that is higher than the first conveying speed value V3a as previously described. Accordingly, even if the target tape continuous body 3a is a material that has poor ease-of-cutting, such a continuous body 3a can be cut reliably. This will be described in detail below.

First, as a comparative example, if the speed value Vc in the cutting even speed region is set to the same value as the first conveying speed value V3a, the target tape continuous body 3a is substantially not subjected to the tensile force F' (FIG. 4) in the rotation directions Dc21 and Dc31 from the receiving blade 23 and the cutter blade 33. Accordingly, due to the fact that the target tape continuous body 3a is a material that has poor ease-of-cutting, if a portion fails to be cut by clamping between the receiving blade 23 and the cutter blade 33, the tensile force F' cannot compensate for the failure to cut that portion. As a result, the target tape continuous body 3a may fail to be cut. However, in light of this, if the speed value Vc in the cutting even speed region is set higher than the first conveying speed value V3a as described above, the receiving blade 23 and the cutter blade 33 can apply an appropriate amount of tensile force F' to the target tape continuous body 3a during cutting. Accordingly, even if a portion fails to be cut by clamping, that uncut portion can be cut by the tensile force F' . As a result, even if the target tape continuous body 3a is a material that has poor ease-of-cutting, such a continuous body 3a can be cut reliably.

Also, in this example, the speed value Vc in the cutting even speed region is set to a speed value that is higher than the arithmetic mean of the first conveying speed value V3a and the second conveying speed value V5a. In other words, the speed value Vc in the cutting even speed region shown in FIG. 5A is closer to the second conveying speed value V5a than to the first conveying speed value V3a.

Accordingly, when the target tape continuous body 3a is cut, the continuous body 3a can be reliably subjected to the tensile force F' in the rotation directions Dc21 and Dc31 from the receiving blade 23 and the cutter blade 33. As a result, even if the target tape continuous body 3a is a material that has poor ease-of-cutting, such a continuous body 3a can be cut reliably.

It should be noted that there is no limitation whatsoever to this. Specifically, if the material of the continuous body 3 is a material that has good ease-of-cutting, the speed value Vc in the cutting even speed region may be set lower than the arithmetic mean of the first conveying speed value V3a and the second conveying speed value V5a. In other words, the speed value Vc in the cutting even speed region may be set to a value that is closer to the first conveying speed value V3a than to the second conveying speed value V5a.

According to this configuration, it is possible to reliably reduce the difference between the first conveying speed value V3a of the target tape continuous body 3a and the speed values V23 and V33 of the receiving blade 23 and the cutter blade 33 when the target tape continuous body 3a is cut. Accordingly, it is possible to reliably reduce the tensile force F' (FIG. 4) in the rotation direction Dc21 and Dc31 that can be applied to the target tape continuous body 3a with the receiving blade 23 and the cutter blade 33 during cutting, thereby making it possible to reliably improve stability during cutting.

Also, the cut processing position S31 is included in the cutting even speed region as described above, and in this example, based on the fact that the cutting even speed region has a predetermined size in the rotation directions Dc21 and Dc31 as shown in FIG. 5A, the entire process from start to end of the cutting of the target tape continuous body 3a with the receiving blade 23 and the cutter blade 33 is performed in this even speed region. Accordingly, throughout the duration from start to end of the cutting of the continuous body 3a, the speed values V23 and V33 (mpm) in the rotation directions Dc21 and Dc31 of the receiving blade 23 and the cutter blade 33 are kept constant, thus making it possible to stably cut the continuous body 3a. It should be noted that the "start of cutting" referred to here means "when the cutter blade 33 starts to come into contact with the target tape continuous body 3a".

It should be noted that there is no limitation whatsoever to the above description. Specifically, at least either the start or the end of cutting may be performed at a position that is outside of the cutting even speed region. However, in this case, the effect of improving stability in cutting is reduced commensurately.

Also, as described above, the transfer processing position S43 is included in the transfer even speed region, and in this example, based on the fact that the transfer even speed region has a predetermined size in the rotation direction Dc21 as shown in FIG. 5A, the entire process from start to end of the transfer of the target tape 3 to the exterior sheet continuous web 5a is performed in this even speed region. Accordingly, throughout the duration from start to end of the transfer of the target tape 3, the rotation speed value V21 of the anvil roll 21, which is the speed value V23 (mpm) in the rotation direction Dc21 of the receiving blade 23, is kept constant. Accordingly, the target tape 3, which is held in a non-sliding manner on the outer circumferential surface 21a of the anvil roll 21, can be stably transferred to the exterior sheet continuous web 5a. It should be noted that the "start of transfer" referred to here means "when the target tape 3 starts to come into contact with the continuous web 5a", and the "end of transfer" means "when the entirety of the piece of target tape 3 has completely separated from the outer circumferential surface 21a of the anvil roll 21".

It should be noted that there is no limitation whatsoever to this. Specifically, at least either the start or the end of transfer may be performed at a position that is outside of the transfer even speed region. However, in this case, the effect of improving stability in transfer is reduced commensurately.

It is described above that the cutter roll 31 rotates in coordination with the anvil roll 21, and this coordination is realized with the rolls 21 and 31 each rotating based on a synchronization signal.

Here, the "synchronization signal" is a signal obtained by the repeated output of a unit signal, and the unit signals are rotation angle signals that correspond to rotation angle values from 0° to 360°, for example. Also, in order to produce individual diapers, the cutter roll 31, the anvil roll 21, the target tape conveying mechanism 11, and the continuous web conveying mechanism 40 have respective individual processing operations that constitute a pattern that is repeatedly performed for portions of a diaper.

For example, in this example, the cutter roll 31 and the anvil roll 21 respectively have one cutter blade 33 and one receiving blade 23. Accordingly, the rolls 31 and 21 each perform one rotation operation corresponding to one full rotation as an individual processing operation. Also, as the individual processing operation, the target tape conveying mechanism 11 has an operation for conveying one target tape continuous body 3a having a length L3 (see FIGS. 2A and 2B) in the conveying direction of the target tape 3, and, as the individual processing operation, the continuous web conveying mechanism 40 has an operation for conveying the continuous web 5a by an amount corresponding to the product pitch P1 in the conveying direction.

These individual processing operations and the unit signal are associated with each other in substantially one-to-one correspondence through controlling the positions and speeds of the servomotors described above.

Accordingly, each time a unit signal of the synchronization signal is output, the cutter roll 31 and the anvil roll 21 each rotate one time, the target tape conveying mechanism 11 conveys the target tape continuous body 3a by an amount corresponding to the length L3, and the continuous web conveying mechanism 40 conveys the continuous web 5a by an amount corresponding to the product pitch P1. Accordingly, each time a unit signal is output, one rectangular piece of target tape 3 having the length L3 is cut away from the target tape continuous body 3a, and that piece of target tape 3 is bonded to the continuous web 5a in accordance with the product pitch P1. In other words, the intermediate product 1a is produced with a length corresponding to the product pitch P1, which is a length corresponding to one diaper.

Note that, synchronization problems can arise between the servomotor of the anvil roll 21 and the servomotor of the cutter roll 31 for various reasons, and such synchronization problems can cause a problem in which the receiving blade 23 and the cutter blade 33 become shifted in the rotation direction Dc21 from the relative positions that they are originally to be located at. In this case, there is a risk that the cutter blade 33 is not reliably received by the receiving blade 23.

However, to address this, in this example, the receiving blade 23 of the anvil roll 21 has the arc surface 23a as previously described with reference to FIG. 3, and the arc surface 23a extends along the rotation direction Dc21 of the anvil roll 21 and has a predetermined length in the rotation direction Dc21. Also, this predetermined length is set in consideration of an envisioned deviation amount between the relative positions in the rotation direction Dc21 when a synchronization problem occurs, and therefore the predetermined length is higher in the rotation direction Dc21 than the envisioned deviation amount. Accordingly, even if a synchronization problem occurs and the receiving blade 23 and the cutter blade 33 become shifted in the rotation direction Dc21 from the relative positions that they are originally to be located at, the receiving blade 23 can receive the cutter blade 33 at the arc surface 23a with no problem whatsoever so as to perform cutting. Accordingly, in this case as well, the target tape continuous body 3a can be cut without a big problem occurring.

Also, in the example in FIG. 3, the adhesive application device 15 located in the vicinity of the target tape conveying mechanism 11 applies an adhesive to the surface of the target tape continuous body 3a that faces the cutter blade 33 of the cutter roll 31, of the two surfaces, and here, if the first conveying speed value V3a of the continuous body 3a and the speed value Vc in the cutting even speed region are in the previously described magnitude relationship, it is possible to suppress the attachment of the adhesive to the cutter blade 33 that protrudes from the outer circumferential surface 31a of the cutter roll 31.

Specifically, in this example, as shown in FIG. 5A, the speed value Vc in the cutting even speed region, that is to say the speed values V23 and V33 of the receiving blade 23 and the cutter blade 33 in this even speed region, are set higher than the first conveying speed value V3a of the target tape continuous body 3a. For this reason, compared with the comparative example in which the speed values V23 and V33 of the receiving blade 23 and the cutter blade 33 are set to the same value as the first conveying speed value V3a, it is possible to reduce the time for which the cutter blade 33 is in contact with the adhesive on the continuous body 3a during the cutting of the target tape continuous body 3a, compared to the comparative example, thus making it possible to suppress the attachment of the adhesive to the cutter blade 33. As a result, it is possible to prevent the adhesive from becoming deposited on the cutter blade 33 and reducing the cutting performance of the cutter blade 33. It is also possible to prevent the case where the adhesive deposited on the cutter blade 33 attaches to the cut and produced piece of target tape 3 and soils it.

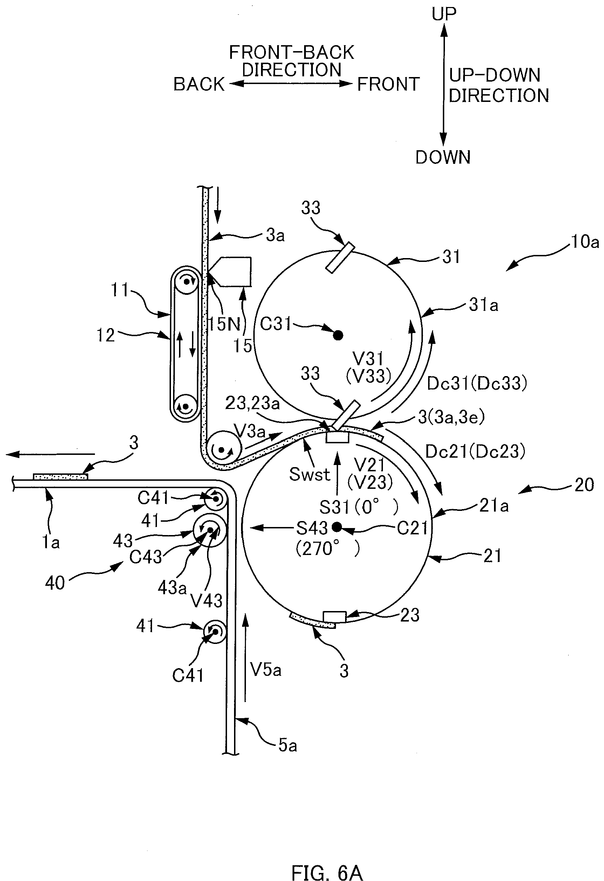

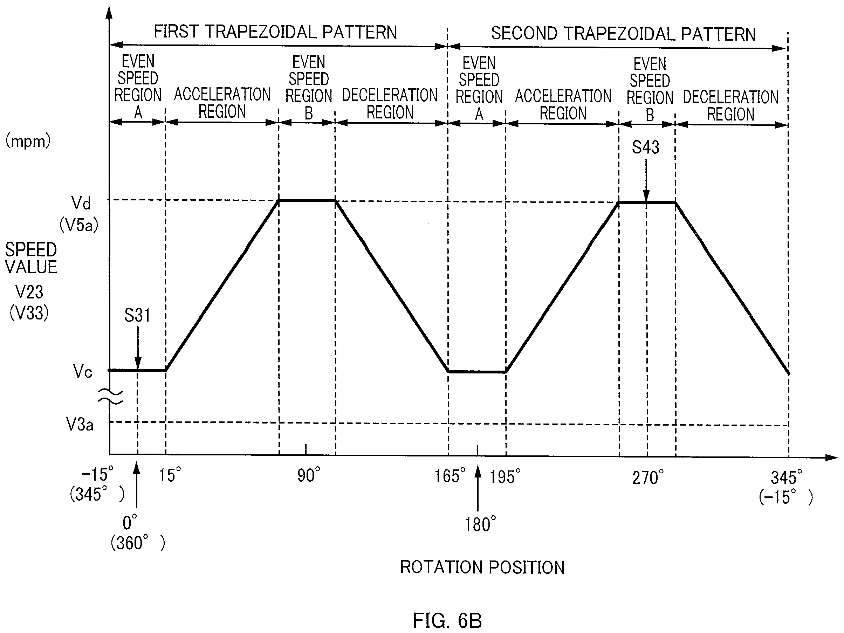

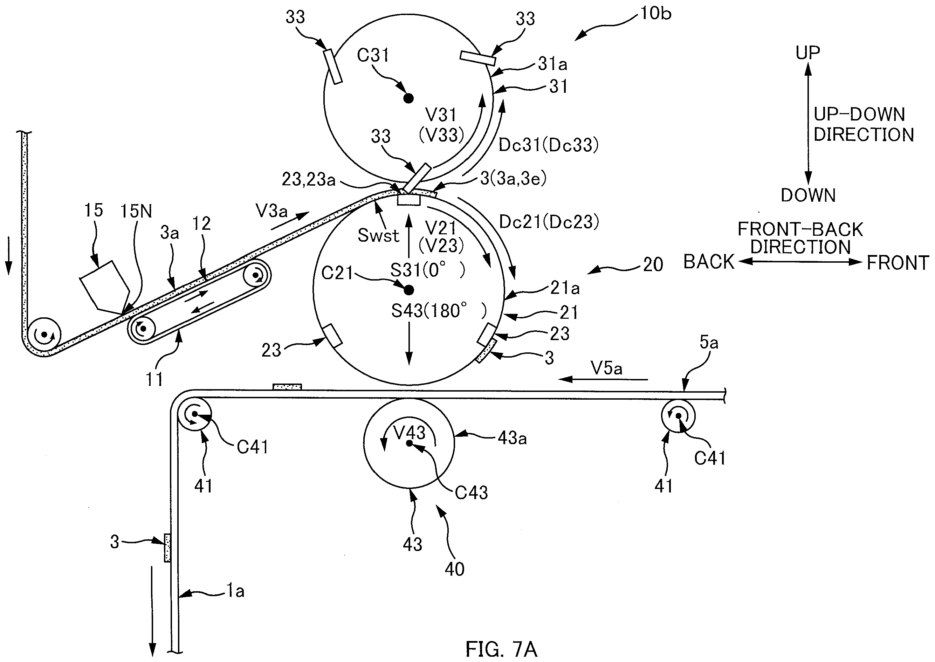

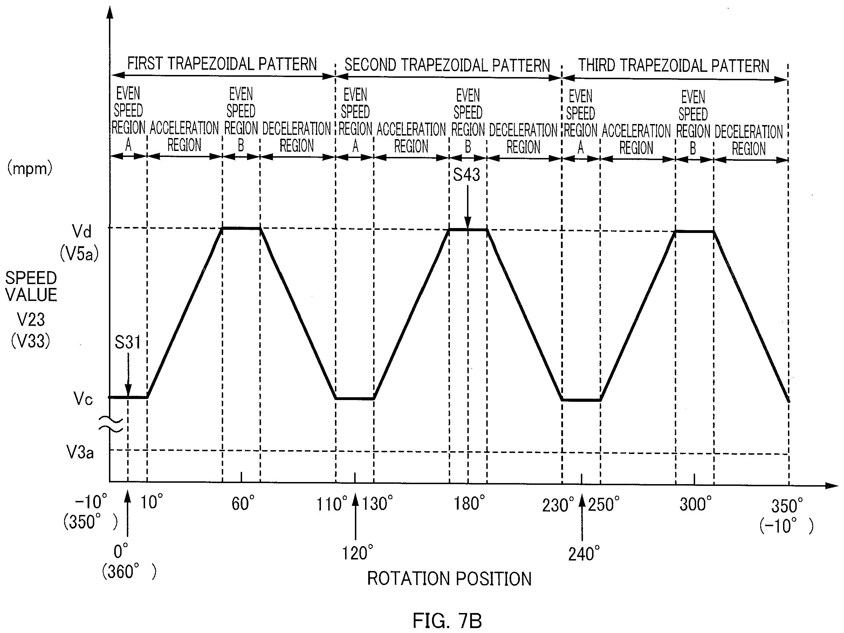

FIG. 6A is a schematic side view of a manufacturing apparatus 10a of a first variation, and FIG. 6B is a diagram illustrating a speed pattern used in the apparatus 10a. Also, FIG. 7A is a schematic side view of a manufacturing apparatus 10b of a second variation, and FIG. 7B is a diagram illustrating a speed pattern used in the apparatus 10b.

In the embodiment described above, there is one cutter blade 33 arranged on the cutter roll 31 and one receiving blade 23 arranged on the anvil roll 21 as shown in FIG. 3, but in the first variation shown in FIG. 6A, two cutter blades 33 and two receiving blades 23 are provided as an example of the case of multiple blades, and in the second variation in FIG. 7A, three cutter blades 33 and three receiving blades 23 are provided. This aspect is the main difference from the embodiment described above. For this reason, the configurations other than this difference are substantially the same as in the embodiment described above. Accordingly, the following mainly describes this difference, and like configurations are denoted by like reference signs and will not be described.

As shown in FIG. 6A, in the first variation, two cutter blades 33 and two receiving blades 23 are provided. Specifically, two cutter blades 33 are provided on the outer circumferential surface 31a of the cutter roll 31 at a uniform pitch of 180° in the rotation direction Dc31, and two receiving blades 23 are provided on the outer circumferential surface 21a of the anvil roll 21 at a uniform pitch of 180° in the rotation direction Dc21. Furthermore, the arrangement pitch (m) in the rotation direction Dc31 of the cutter blade 33 defined above, that is to say the arrangement pitch (m) of the cutter blade 33 in the rotation direction Dc31 at the position of the blade tip of the cutter blade 33, is set to the same value as the arrangement pitch (m) in the rotation direction Dc21 of the receiving blade 23, that is to say the arrangement pitch (m) of the receiving blade 23 in the rotation direction Dc21 at the position of the arc surface 23a of the receiving blade 23.

Accordingly, at the cut processing position S31, which is the 0° rotation position of the anvil roll 21, the cutter blades 33 and the receiving blades 23 face each other at the rate of one time each one-half rotation of the rolls 31 and 21, and therefore one piece of target tape 3 is cut away on the outer circumferential surface 21a of the anvil roll 21 each time it rotates one-half revolution. The cut-away piece of target tape 3 is held in a non-sliding manner on the outer circumferential surface 21a of the anvil roll 21, and is conveyed to the transfer processing position S43 at the 270° rotation position by rotation of the roll 21. At the position S43, it is bonded and transferred to the exterior sheet continuous web 5a that is being conveyed at the second conveying speed value V5a.

It should be noted that the transfer processing position S43 is not limited in any way to being the 270° rotation position described above, and the transfer processing position S43 can be set to any rotation position that satisfies the following condition. Specifically, the transfer processing position S43 can be set to any position in the rotation direction Dc21 of the anvil roll 21 that is a position at which, when one of the two receiving blades 23 passes the cut processing position S31, the other receiving blade 23 is not located. For example, the transfer processing position S43 can be set to the 90° rotation position as well.

If the transfer processing position S43 is set to this position, the speed value V23 of the receiving blade 23 when passing the cut processing position S31 and the speed value V23 of the receiving blade 23 when passing the transfer processing position S43 can be reliably set differently from each other. Accordingly, the speed pattern of the anvil roll 21 can be easily set such that the speed value V23 (Vc) of the receiving blade 23 when passing the cut processing position S31 is lower than the speed value V23 (Vd) of the receiving blade 23 when passing the transfer processing position S43.

Also, as shown in FIG. 6B, the speed pattern used in this first variation has an even arrangement of two identical trapezoidal patterns in the range of -15° (345°) to 345° (-15°) in the rotation directions Dc21 and Dc31 of the anvil roll 21 and the cutter roll 31. Specifically, the speed pattern has a first trapezoidal pattern between the -15° (345°) rotation position and the 165° rotation position in the rotation directions Dc21 and Dc31, and has a second trapezoidal pattern between the 165° rotation position and the 345° (-15°) rotation position. These trapezoidal patterns each have two even speed regions A and B in which the speed values V23 and V33 are mutually different. Also, the cut processing position S31 is included in the even speed region A having a lower speed value in the first trapezoidal pattern, and the transfer processing position S43 is included in the even speed region B having a higher speed value in the second trapezoidal pattern.

For this reason, similarly to the embodiment described above, at the cut processing position S31, the receiving blade 23 and the cutter blade 33 cut the target tape continuous body 3a while moving in the rotation directions Dc21 and Dc31 at the speed value Vc that is lower than the second conveying speed value V5a. Accordingly, the continuous body 3a can be cut while suppressing the tensile force F' (FIG. 4) in the rotation directions Dc21 and Dc31. On the other hand, at the transfer processing position S43, the receiving blade 23 moves at the speed value Vd that is the same as the second conveying speed value V5a. Accordingly, the target tape 3 can be smoothly transferred to the exterior sheet continuous web 5a that is likewise being conveyed to the position S43 at the second conveying speed value V5a.

It should be noted that in the first variation as well, the cutter roll 31 and the anvil roll 21 rotate in coordination with each other based on a synchronization signal. Also note that in the first variation, the servomotors are controlled in terms of position and speed such that the rolls 31 and 21 rotate one-half revolution each time a unit signal of the synchronization signal is output. For this reason, the cutter roll 31 rotates such that when one of the two receiving blades 23 of the anvil roll 21 passes the cut processing position S31 in the rotation direction Dc21 due to rotation of the roll 21, one of the two cutter blades 33 of the cutter roll 31 faces that receiving blade 23, and when the other receiving blade 23 passes the cut processing position S31, the other cutter blade 33 faces that other receiving blade 23. Accordingly, the cutter blade 33 and the receiving blade 23 that correspond to each other clamp the target tape continuous body 3a together and cut it to produce a piece of target tape 3.

Also, in the second variation in FIG. 7A, three cutter blades 33 and three receiving blades 23 are provided. Specifically, three cutter blades 33 are provided on the outer circumferential surface 31a of the cutter roll 31 at a uniform pitch of 120° in the rotation direction Dc31, and three receiving blades 23 are provided on the outer circumferential surface 21a of the anvil roll 21 at a uniform pitch of 120° in the rotation direction Dc21. Furthermore, the arrangement pitch (m) in the rotation direction Dc31 of the cutter blade 33 defined above, that is to say the arrangement pitch (m) of the cutter blade 33 in the rotation direction Dc31 at the position of the blade tip of the cutter blade 33, is set to the same value as the arrangement pitch (m) in the rotation direction Dc21 of the receiving blade 23, that is to say the arrangement pitch (m) of the receiving blade 23 in the rotation direction Dc21 at the position of the arc surface 23a of the receiving blade 23.

Accordingly, at the cut processing position S31, which is the 0° rotation position of the anvil roll 21, the cutter blades 33 and the receiving blades 23 face each other at the rate of one time each one-third rotation of the rolls 31 and 21, and therefore one piece of target tape 3 is cut away on the outer circumferential surface 21a of the anvil roll 21 each time it rotates one-third revolution. The cut-away piece of target tape 3 is held in a non-sliding manner on the outer circumferential surface 21a of the anvil roll 21, and is conveyed to the transfer processing position S43 at the 180° rotation position by rotation of the roll 21. At the position S43, it is bonded and transferred to the exterior sheet continuous web 5a that is being conveyed at the second conveying speed value V5a.

Note that in the second variation as well, the transfer processing position S43 is not limited in any way to being the 180° rotation position described above, and similarly to the first variation, the transfer processing position S43 can be set to any rotation position that satisfies the following condition. Specifically, the transfer processing position S43 can be set to any position in the rotation direction Dc21 of the anvil roll 21 that is a position at which, when one of the three receiving blades 23 passes the cut processing position S31, the other two receiving blades 23 are not located. For example, the transfer processing position S43 can be set to the 90° or 270° rotation position.

Also, as shown in FIG. 7B, the speed pattern used in this second variation has an even arrangement of three identical trapezoidal patterns in the range of -10° (350°) to 350° (-10°) in the rotation directions Dc21 and Dc31 of the anvil roll 21 and the cutter roll 31. Specifically, the speed pattern has a first trapezoidal pattern between the -10° (350°) rotation position and the 110° rotation position in the rotation directions Dc21 and Dc31, has a second trapezoidal pattern between the 110° rotation position and the 230° rotation position, and has a third trapezoidal pattern between the 230° rotation position and the 350° (-10°) rotation position.

These trapezoidal patterns each have two even speed regions A and B in which the speed values V23 and V33 are mutually different. Also, the cut processing position S31 is set in the even speed region A having a lower speed value in the first trapezoidal pattern, and the transfer processing position S43 is set in the even speed region B having a higher speed value in the second trapezoidal pattern.

For this reason, similarly to the embodiment described above, at the cut processing position S31, the receiving blade 23 and the cutter blade 33 moving in the rotation directions Dc21 and Dc31 at the speed value Vc that is lower than the second conveying speed value V5a, and can thus stably cut the target tape continuous body 3a. On the other hand, at the transfer processing position S43, the receiving blade 23 moves at the speed value Vd that is the same as the second conveying speed value V5a. Accordingly, the target tape 3 can be smoothly transferred to the exterior sheet continuous web 5a that is likewise being conveyed to the position S43 at the second conveying speed value V5a.

Also, in the second variation as well, the cutter roll 31 and the anvil roll 21 rotate in coordination with each other based on a synchronization signal. Note that the servomotors are controlled in terms of position or speed such that the rolls 31 and 21 rotate one-third revolution each time a unit signal of the synchronization signal is output. For this reason, the cutter roll 31 rotates such that when the first one of the three receiving blades 23 of the anvil roll 21 passes the cut processing position S31 in the rotation direction Dc21 due to rotation of the roll 21, the first one of the three cutter blades 33 of the cutter roll 31 faces that first receiving blade 23, when the second receiving blade 23 passes the cut processing position S31, the second cutter blade 33 faces the second receiving blade 23, and when the third receiving blade 23 passes the cut processing position S31, the third cutter blade 33 faces the third receiving blade 23. Accordingly, the cutter blade 33 and the receiving blade 23 that correspond to each other clamp the target tape continuous body 3a together and cut it to produce a piece of target tape 3.

The first and second variations have been described above as examples of cases where multiple cutter blades 33 and multiple receiving blades 23 are provided, but the number of cutter blades 33 and receiving blades 23 that are provided is not limited whatsoever to being two or three. In other words, four or more cutter blades 33 and four or more receiving blades 23 may be provided.

### Other Embodiments

A description has been given of the embodiment of the present invention. The foregoing embodiment is for facilitating the understanding of the present invention and is not to be construed as limiting the present invention. The present invention may be modified and/or improved without departing from the gist thereof, and it goes without saying that the present invention encompasses any equivalents thereof. For example, the following modifications are possible.

In the above embodiments, the target tape continuous body 3a is shown as an example of the first continuous sheet, and the exterior sheet continuous web 5a is shown as an example of the second continuous sheet, but there is no limitation whatsoever to this. For example, the first continuous sheet may be a continuous body of the previously mentioned fastening tape, or may be a continuous sheet of the previously mentioned leak-proof sheet. Also, the second continuous sheet may be a continuous sheet of the previously mentioned top face sheet. Furthermore, the material of the first continuous sheet is not limited whatsoever to being a nonwoven fabric, and may be a woven fabric, a film, or the like, and similarly, the material of the second continuous sheet is not limited whatsoever to being a nonwoven fabric, and may be a woven fabric, a film, or the like.

Although a disposable diaper is shown as an example of the absorbent article in the above embodiments, there is no limitation whatsoever to this, and it may be any article that absorbs excreted fluid from the wearer. For example, the absorbent article may be a sanitary napkin or a urine absorbing pad.

Although the first conveying speed value V3a (mpm) of the target tape continuous body 3a, which is the first continuous sheet, is kept constant in the above embodiments, there is no limitation whatsoever to this. For example, the first conveying speed value V3a may vary in a cyclic manner, for example. It should be noted that in this case, the tensile force F' (FIG. 4) applied to the continuous body 3a can increase in an oscillating manner, and as a result, there is a risk of cutting becoming unstable and the continuous body 3a becoming damaged. For this reason, it is desirable that the first conveying speed value V3a is kept constant as described above.

Although the second conveying speed value V5a (mpm) of the exterior sheet continuous web 5a, which is the second continuous sheet, is kept constant in the above embodiments, there is no limitation whatsoever to this. For example, the second conveying speed value V5a may vary in a cyclic manner, for example. It should be noted that in this case, there is a risk of the transfer of the target tape 3 to the continuous web 5a becoming unstable. For this reason, it is desirable that the second conveying speed value V5a is kept constant as described above.

In the above embodiments, the cutting even speed region is set so as to include the cut processing position S31 for each of the rotation directions Dc21 and Dc31 of the anvil roll 21 and the cutter roll 31. Accordingly, in the regions that are adjacent to the cut processing position S31 on the upstream side or the downstream side in the rotation directions Dc21 and Dc31, the speed values V23 and V33 of the receiving blade 23 and the cutter blade 33 are kept constant, but there is no limitation whatsoever to this. In other words, the speed values V23 and V33 of the receiving blade 23 and the cutter blade 33 may vary in the adjacent regions on the upstream side or the downstream side. Note that in this case, there is a risk of the cutting becoming unstable. For this reason, it is desirable that the even speed regions are set as described above.

In the above embodiments, the transfer even speed region is set so as to include the transfer processing position S43 for the rotation direction Dc21 in the anvil roll 21. Accordingly, the speed value V23 of the receiving blade 23 is kept constant in the region that is adjacent to the transfer processing position S43 on the upstream side or the downstream side in the rotation direction Dc21, but there is no limitation whatsoever to this. In other words, the speed value V23 of the receiving blade 23 may vary in the adjacent region on the upstream side or the downstream side. It should be noted that in this case, there is a risk of the transfer of the target tape 3 to the continuous web 5a becoming unstable. For this reason, it is desirable that the even speed regions are set as described above.

Although trapezoidal patterns are described as examples of the speed pattern as shown in FIG. 5A in the above embodiments, there is no limitation whatsoever to this. For example, in the case where there is no problem if an even speed region is not set, it is possible to use a waveform pattern formed by curved lines, such as a sinusoidal wave pattern. Furthermore, a waveform pattern including a combination of a trapezoidal pattern and a sinusoidal pattern may be used, or a pattern having other shapes may be used.

As shown in FIG. 3, the arc surface 23a of the receiving blade 23 of the anvil roll 21 is in a flush relationship with the outer circumferential surface 21a of the anvil roll 21 in the above embodiments, but there is no limitation whatsoever to this. Specifically, the arc surface 23a of the receiving blade 23 may protrude outward in the radius of rotation direction Dr21 of the roll 21 beyond the outer circumferential surface 21a, or may be recessed inward. Also, although the surface 23a of the receiving blade 23 that faces outward in the radius of rotation direction Dr21 is an arc surface in the above embodiments, it is not limited whatsoever to being an arc surface. For example, it may be a flat surface that extends along the rotation direction Dc21. It should be noted that in the case of being a flat surface, there is a risk that the receiving blade 23 cannot receive the cutter blade 33 if a synchronization problem occurs in the rotation operations described above, and in such as case, cutting may become unstable. Therefore, it is desirable that this surface is an arc surface as described above.

Although the unit signal of the synchronization signal is a signal indicated by rotation angle values from 0° to 360° in the above embodiments, there is no limitation whatsoever to this. For example, the unit signal of the synchronization signal may be configured by digital values from 0 to 8191, for example.

In the above embodiments, the continuous web conveying mechanism 40 has the roll 43 that corresponds to the transfer processing position S43 as shown in FIG. 3, but the roll 43 is not essential. In other words, the roll 43 can be omitted as long as the target tape 3 on the outer circumferential surface 21a of the anvil roll 21 can be transferred to the continuous web 5a in the transfer processing position S43. It should be noted that from the viewpoint of stability in transfer, it is desirable to provide the roll 43.

In the above embodiment, based on the fact that, for example, one cutter blade 33 and one receiving blade 23 are provided as shown in FIG. 3, it is described that "the arrangement pitches (m) in the rotation directions Dc21 and Dc31 of the receiving blade 23 and the cutter blade 33 are the circumferential lengths (m) of one revolution around the revolving paths of the receiving blade 23 and the cutter blade 33, and these circumferential lengths are the same values as each other". However, although the circumferential lengths are not described in further detail, given that the blades 23 and 33 rotate according to the speed pattern in FIG. 5A, the circumferential lengths are shorter than the product pitch P1 such that pieces of the target tape 3 are arranged side-by-side in the rotation direction Dc21 at the previously described product pitch P1 (FIGS. 2A and 2B) . It should be noted that there is no limitation whatsoever to this. For example, it is possible to use a speed pattern in which, in the acceleration region of the speed pattern in FIG. 5A, the speed values V23 and V33 of the blades 23 and 33 of the rolls 21 and 31 are first set higher than the speed value Vd in the transfer even speed region, and then lowered so as to reach the speed value Vd immediately before the transfer even speed region, and in this case, the aforementioned circumferential lengths can be set to the same value as the product pitch P1.

### List of Reference Numerals

1 back-face sheet, 1a intermediate product (composite body of continuous sheets)
3 target tape (cutform sheet),
3a target tape continuous body (first continuous sheet),
3e leading end portion,
5 exterior sheet, 5a exterior sheet continuous web (second continuous sheet),
10 slip cutting device (manufacturing apparatus), 10a manufacturing apparatus, 10b manufacturing apparatus,
11 target tape conveying mechanism (first conveying mechanism), 12 endless belt,
15 adhesive application device, 15N nozzle,
20 anvil roll mechanism (holding roll mechanism),
21 anvil roll (holding roll), 21a outer circumferential surface,
23 receiving blade, 23a arc surface,
31 cutter roll, 31a outer circumferential surface, 33 cutter blade,
40 continuous web conveying mechanism, 41 roll, 43 roll, 43a outer
circumferential surface,
C21 rotation axis, C31 rotation axis, C41 rotation axis, C43 rotation axis,
S31 cut processing position (arrangement position, first predetermined position),
S43 transfer processing position (second predetermined position), Swst wrapping start position

## Claims

1. A method for manufacturing a composite body of continuous sheets pertaining to an absorbent article, in which a first continuous sheet is cut to produce a cutform sheet, and the cutform sheet is bonded and transferred to a second continuous sheet while forming an interval on the second continuous sheet between the cutform sheet and another cutform sheet that is adjacent thereto in a continuity direction of the second continuous sheet, the method comprising:
conveying the first continuous sheet at a first conveying speed value (mpm) in a conveying direction that is a continuity direction of the first continuous sheet;
rotating a holding roll with use of a motor in a rotation direction that conforms to the conveying direction of the first continuous sheet, the holding roll being rotated while the first continuous sheet is held on an outer circumferential surface of the holding roll such that the first continuous sheet slides upstream in the rotation direction on the outer circumferential surface;
rotating a cutter roll with use of a motor separate from the motor for the holding roll, the cutter roll being arranged in a first predetermined position in the rotation direction of the holding roll and being rotated in a rotation direction that conforms to the conveying direction;
cutting and producing the cutform sheet with a receiving blade in the outer circumferential surface of the holding roll cutting the first continuous sheet in conjunction with a cutter blade of the cutter roll when the receiving blade passes the first predetermined position in the rotation direction of the holding roll; and
transferring the cutform sheet on the outer circumferential surface to the second continuous sheet and bonding the cutform sheet on the second continuous sheet when the cutform sheet held in a non-sliding manner on the outer circumferential surface passes a second predetermined position in the rotation direction, the second continuous sheet being conveyed toward the second predetermined position at a second conveying speed value (mpm) that is higher than the first conveying speed value (mpm),
wherein the number of receiving blades in the rotation direction on the holding roll and the number of cutter blades in the rotation direction on the cutter roll are identical,
an arrangement pitch in the rotation direction of the receiving blade on a revolving path drawn with the receiving blade due to rotation of the holding roll in the rotation direction is identical to an arrangement pitch in the rotation direction of the cutter blade in a revolving path drawn with the cutter blade due to rotation of the cutter roll in the rotation direction,
the holding roll rotates based on a defined speed pattern such that a speed value (mpm) in the rotation direction of the receiving blade when passing the second predetermined position is identical to the second conveying speed value, and such that a speed value (mpm) in the rotation direction of the receiving blade when passing the first predetermined position is lower than the speed value (mpm) in the rotation direction of the receiving blade when passing the second predetermined position, and
in the cutting and producing, the speed value (mpm) in the rotation direction of the receiving blade when passing the first predetermined position is higher than the first conveying speed value of the first continuous sheet.

2. The method for manufacturing a composite body of continuous sheets pertaining to an absorbent article according to claim 1,
wherein the second conveying speed value of the second continuous sheet is kept constant, and
in the transferring, the speed value (mpm) in the rotation direction of the receiving blade is kept constant from a start to an end of transfer of the cutform sheet to the second continuous sheet.

3. The method for manufacturing a composite body of continuous sheets pertaining to an absorbent article according to claim 1 or 2, wherein the speed value (mpm) in the rotation direction of the receiving blade when passing the first predetermined position is higher than an arithmetic mean of the first conveying speed value and the second conveying speed value.

4. The method for manufacturing a composite body of continuous sheets pertaining to an absorbent article according to claim1 or 2, wherein the speed value (mpm) in the rotation direction of the receiving blade when passing the first predetermined position is lower than an arithmetic mean of the first conveying speed value and the second conveying speed value.

5. The method for manufacturing a composite body of continuous sheets pertaining to an absorbent article according to any of claims 1 to 4,
wherein the cutter blade protrudes from an outer circumferential surface of the cutter roll and
an adhesive for bonding of the second continuous sheet and the cutform sheet is applied to, out of two surfaces of the first continuous sheet, a surface that faces the cutter blade.

6. The method for manufacturing a composite body of continuous sheets pertaining to an absorbent article according to any of claims 1 to 5, wherein in the cutting and producing, the speed value (mpm) in the rotation direction of the receiving blade is kept constant from a start to an end of cutting of the first continuous sheet with the cutter blade and the receiving blade.

7. The method for manufacturing a composite body of continuous sheets pertaining to an absorbent article according to any of claims 1 to 6, wherein the first conveying speed value of the first continuous sheet is kept constant.

8. The method for manufacturing a composite body of continuous sheets pertaining to an absorbent article according to any of claims 1 to 7,
wherein the receiving blade is a member separate from the holding roll,
the receiving blade has an arc surface that extends along the rotation direction of the holding roll, and
the first continuous sheet is clamped between and cut with the arc surface and the cutter blade.

9. The method for manufacturing a composite body of continuous sheets pertaining to an absorbent article according to any of claims 1 to 8,
wherein a plurality of receiving blades and a plurality of cutter blades are provided, and
the second predetermined position is set in a position in the rotation direction of the holding roll that is a position in which, when one of the plurality of receiving blades passes the first predetermined position, another one of the receiving blades is not located.

10. An apparatus for manufacturing a composite body of continuous sheets pertaining to an absorbent article, in which a first continuous sheet is cut to produce a cutform sheet, and the cutform sheet is bonded and transferred to a second continuous sheet while forming an interval on the second continuous sheet between the cutform sheet and another cutform sheet that is adjacent thereto in a continuity direction of the second continuous sheet, the apparatus comprising:
a first conveying mechanism that conveys the first continuous sheet at a first conveying speed value (mpm) in a conveying direction that is a continuity direction of the first continuous sheet;
a holding roll mechanism having a holding roll that is driven to rotate with use of a motor in a rotation direction that conforms to the conveying direction of the first continuous sheet, the holding roll mechanism rotating the holding roll while the first continuous sheet is held on an outer circumferential surface of the holding roll such that the first continuous sheet slides upstream in the rotation direction on the outer circumferential surface; and
a cutter roll that is arranged in a first predetermined position in the rotation direction of the holding roll, the cutter roll being driven to rotate in a rotation direction that conforms to the conveying direction, with use of a motor separate from the motor for the holding roll,
wherein the cutform sheet is produced by a receiving blade at the outer circumferential surface of the holding roll cutting the first continuous sheet in conjunction with a cutter blade of the cutter roll, in the speed value (mpm) in the rotation direction higher than the first conveying speed value of the first continuous sheet, when the receiving blade passes the first predetermined position in the rotation direction of the holding roll,
the cutform sheet on the outer circumferential surface is bonded and transferred to the second continuous sheet when the cutform sheet held in a non-sliding manner on the outer circumferential surface passes a second predetermined position in the rotation direction, the second continuous sheet being conveyed toward the second predetermined position at a second conveying speed value (mpm) that is higher than the first conveying speed value (mpm),
the number of receiving blades in the rotation direction on the holding roll and the number of cutter blades in the rotation direction on the cutter roll are identical,
an arrangement pitch in the rotation direction of the receiving blade on a revolving path drawn with the receiving blade due to rotation of the holding roll in the rotation direction is identical to an arrangement pitch in the rotation direction of the cutter blade in a revolving path drawn with the cutter blade due to rotation of the cutter roll in the rotation direction, and
the holding roll rotates based on a defined speed pattern such that a speed value (mpm) in the rotation direction of the receiving blade when passing the second predetermined position is identical to the second conveying speed value, and such that a speed value (mpm) in the rotation direction of the receiving blade when passing the first predetermined position is lower than the speed value (mpm) in the rotation direction of the receiving blade when passing the second predetermined position.

## Patentansprüche

1. Verfahren zur Herstellung eines Verbundkörpers aus kontinuierlichen Lagen, der zu einem absorbierenden Artikel gehört, bei dem eine erste kontinuierliche Lage geschnitten wird um eine zugeschnittene Lage zu erzeugen, und die zugeschnittene Lage mit einer zweiten kontinuierliche Lage verbunden und übertragen wird, während auf der zweiten kontinuierlichen Lage ein Abstand zwischen der zugeschnittenen Lage und einer anderen zugeschnittenen Lage, die in einer Verlaufsrichtung der zweiten kontinuierlichen Lage daran angrenzt, ausgebildet wird, wobei das Verfahren umfasst:
Fördern der ersten kontinuierlichen Lage bei einem ersten Fördergeschwindigkeitswert (mpm) in einer Förderrichtung, die eine Verlaufsrichtung der ersten kontinuierlichen Lage ist;
Rotieren einer Halterolle unter Verwendung eines Motors in einer Rotationsrichtung, die mit der Förderrichtung der ersten kontinuierlichen Lage übereinstimmt, wobei die Halterolle rotiert wird, während die erste kontinuierliche Lage an einer Außenumfangsfläche der Halterolle gehalten wird, so dass die erste kontinuierliche Lage stromaufwärts in der Rotationsrichtung auf der Außenumfangsfläche gleitet;
Rotieren einer Schneiderolle unter Verwendung eines von dem Motor für die Halterolle getrennten Motors, wobei die Schneiderolle in einer ersten vorbestimmten Position in der Rotationsrichtung der Halterolle angeordnet ist und in einer Rotationsrichtung gedreht wird, die mit der Förderrichtung übereinstimmt;
Schneiden und Herstellen der zugeschnittenen Lage mit einer Aufnahmeklinge in der Außenumfangsfläche der Halterolle, wobei die erste kontinuierliche Lage in Verbindung mit einer Schneideklinge der Schneiderolle geschnitten wird, wenn die Aufnahmeklinge die erste vorbestimmte Position in der Rotationsrichtung der Halterolle passiert; und
Übertragen der zugeschnittenen Lage an der Außenumfangsfläche auf die zweite kontinuierliche Lage und Verbinden der zugeschnittenen Lage mit der zweiten kontinuierlichen Lage, wenn die zugeschnittene Lage, die in nicht gleitender Weise an der Außenumfangsfläche gehalten wird, eine zweite vorbestimmte Position in der Rotationsrichtung passiert, wobei die zweite kontinuierliche Lage mit einem zweiten Fördergeschwindigkeitswert (mpm), der höher als der erste Fördergeschwindigkeitswert (mpm) ist, zu der zweiten vorbestimmten Position gefördert wird,
wobei die Anzahl der Aufnahmeklingen in der Rotationsrichtung auf der Halterolle und die Anzahl der Schneideklingen in der Rotationsrichtung auf der Schneiderolle identisch sind,
wobei ein Anordnungsabstand in der Rotationsrichtung der Aufnahmeklinge auf einer mit der Aufnahmeklinge gezogenen Umlaufbahn aufgrund der Rotation der Halterolle in der Rotationsrichtung identisch ist mit einem Anordnungsabstand in der Rotationsrichtung der Schneideklinge auf einer mit der Schneideklinge gezogenen Umlaufbahn aufgrund der Rotation der Schneiderolle in der Rotationsrichtung,
wobei die Halterolle sich auf der Grundlage eines definierten Geschwindigkeitsmusters derart dreht, dass ein Geschwindigkeitswert (mpm) in der Rotationsrichtung der Aufnahmeklinge beim Passieren der zweiten vorbestimmten Position identisch mit dem zweiten Fördergeschwindigkeitswert ist, und derart, dass ein Geschwindigkeitswert (mpm) in der Rotationsrichtung der Aufnahmeklinge beim Passieren der ersten vorbestimmten Position niedriger ist als der Geschwindigkeitswert (mpm) in der Rotationsrichtung der Aufnahmeklinge beim Passieren der zweiten vorbestimmten Position, und
wobei beim Schneiden und Produzieren der Geschwindigkeitswert (mpm) in der Rotationsrichtung der Aufnahmeklinge beim Passieren der ersten vorbestimmten Position höher ist als der erste Fördergeschwindigkeitswert der ersten kontinuierlichen Lage.

2. Verfahren zur Herstellung eines Verbundkörpers aus kontinuierlichen Lagen, der zu einem absorbierenden Artikel gemäß Anspruch 1 gehört,
wobei der zweite Fördergeschwindigkeitswert der zweiten kontinuierlichen Lage konstant gehalten wird, und
bei der Übertragung der Geschwindigkeitswert (mpm) in der Rotationsrichtung der Aufnahmeklinge von einem Anfang bis zu einem Ende der Übertragung der zugeschnittenen Lage auf die zweite kontinuierliche Lage konstant gehalten wird.

3. Verfahren zur Herstellung eines Verbundkörpers aus kontinuierlichen Lagen, der zu einem absorbierenden Artikel gemäß Anspruch 1 oder 2 gehört, wobei der Geschwindigkeitswert (mpm) in der Rotationsrichtung der Aufnahmeklinge beim Passieren der ersten vorbestimmten Position höher ist als ein arithmetisches Mittel aus dem ersten Fördergeschwindigkeitswert und dem zweiten Fördergeschwindigkeitswert.

4. Verfahren zur Herstellung eines Verbundkörpers aus kontinuierlichen Lagen, der zu einem absorbierenden Artikel gemäß Anspruch 1 oder 2 gehört, wobei der Geschwindigkeitswert (mpm) in der Rotationsrichtung der Aufnahmeklinge beim Passieren der ersten vorbestimmten Position niedriger ist als ein arithmetisches Mittel aus dem ersten Fördergeschwindigkeitswert und dem zweiten Fördergeschwindigkeitswert.

5. Verfahren zur Herstellung eines Verbundkörpers aus kontinuierlichen Lagen, der zu einem absorbierenden Artikel gemäß einem der Ansprüche 1 bis 4 gehört,
wobei die Schneideklinge von einer Außenumfangsfläche der Schneiderolle vorsteht, und
ein Klebstoff zum Verbinden der zweiten kontinuierlichen Lage und der zugeschnittenen Lage von zwei Oberflächen der ersten kontinuierlichen Lage auf eine Oberfläche aufgetragen wird, die der Schneideklinge zugewandt ist.

6. Verfahren zur Herstellung eines Verbundkörpers aus kontinuierlichen Lagen, der zu einem absorbierenden Artikel gemäß einem der Ansprüche 1 bis 5 gehört, wobei beim Schneiden und Herstellen der Geschwindigkeitswert (mpm) in der Rotationsrichtung der Aufnahmeklinge von einem Anfang bis zu einem Ende des Schneidens der ersten kontinuierlichen Lage mit der Schneideklinge und der Aufnahmeklinge konstant gehalten wird.

7. Verfahren zur Herstellung eines Verbundkörpers aus kontinuierlichen Lagen, der zu einem absorbierenden Artikel gemäß einem der Ansprüche 1 bis 6 gehört, wobei der erste Fördergeschwindigkeitswert der ersten kontinuierlichen Lage konstant gehalten wird.

8. Verfahren zur Herstellung eines Verbundkörpers aus kontinuierlichen Lagen, der zu einem absorbierenden Artikel gemäß einem der Ansprüche 1 bis 7 gehört,
wobei die Aufnahmeklinge ein von der Halterolle getrenntes Element ist,
die Aufnahmeklinge eine Kreisbogenfläche aufweist, die sich entlang der Rotationsrichtung der Halterolle erstreckt, und
die erste kontinuierliche Lage zwischen der Kreisbogenfläche und der Schneideklinge eingeklemmt und mit der Kreisbogenfläche und der Schneideklinge geschnitten wird.

9. Verfahren zur Herstellung eines Verbundkörpers aus kontinuierlichen Lagen, der zu einem absorbierenden Artikel gemäß einem der Ansprüche 1 bis 8 gehört,
wobei eine Vielzahl von Aufnahmeklingen und eine Vielzahl von Schneideklingen vorgesehen sind, und
die zweite vorbestimmte Position in einer Position in Rotationsrichtung der Halterolle angeordnet ist, die eine Position ist, in der, wenn eine der Vielzahl von Aufnahmeklingen die erste vorbestimmte Position passiert, eine andere der Aufnahmeklingen nicht angeordnet ist.

10. Vorrichtung zum Herstellen eines Verbundkörpers aus kontinuierlichen Lagen, der zu einem absorbierenden Artikel gehört, bei der eine erste kontinuierliche Lage geschnitten wird, um eine zugeschnittene Lage herzustellen, und die zugeschnittene Lage mit einer zweiten kontinuierlichen Lage verbunden und auf diese übertragen wird, während auf der zweiten kontinuierlichen Lage ein Abstand zwischen der zugeschnittenen Lage und einer anderen zugeschnittenen Lage ausgebildet ist, die daran in einer Verlaufsrichtung der zweiten kontinuierlichen Lage angrenzt, wobei die Vorrichtung aufweist:
einen ersten Fördermechanismus, der die erste kontinuierliche Lage mit einem ersten Fördergeschwindigkeitswert (mpm) in einer Förderrichtung fördert, die eine Verlaufsrichtung der ersten kontinuierlichen Lage ist;
einen Halterollenmechanismus mit einer Halterolle, die derart angetrieben ist, dass sie sich unter Verwendung eines Motors in einer Rotationsrichtung dreht, die mit der Förderrichtung der ersten kontinuierlichen Lage übereinstimmt, wobei der Halterollenmechanismus die Halterolle dreht, während die erste kontinuierliche Lage an einer Außenumfangsfläche der Halterolle gehalten ist, so dass die erste kontinuierliche Lage stromaufwärts in der Rotationsrichtung auf der Außenumfangsfläche gleitet; und
eine Schneiderolle, die in einer ersten vorbestimmten Position in der Rotationsrichtung der Halterolle angeordnet ist, wobei die Schneiderolle derart angetrieben ist, dass diese in einer Rotationsrichtung rotiert, die mit der Förderrichtung übereinstimmt, unter Verwendung eines von dem Motor getrennten Motors für die Halterolle,
wobei die zugeschnittene Lage durch eine Aufnahmeklinge an der Außenumfangsfläche der Halterolle ausgebildet ist, welche die erste kontinuierliche Lage in Verbindung mit einer Schneideklinge der Schneiderolle in dem Geschwindigkeitswert (mpm) in der Rotationsrichtung schneidet, der höher als der erste Fördergeschwindigkeitswert der ersten kontinuierlichen Lage ist, wenn die Aufnahmeklinge die erste vorbestimmte Position in der Rotationsrichtung der Halterolle passiert,
wobei die zugeschnittene Lage auf der Außenumfangsfläche verklebt und auf die zweite kontinuierliche Lage übertragen wird, wenn die auf der Außenumfangsfläche nicht gleitend gehaltene zugeschnittene Lage eine zweite vorbestimmte Position in der Rotationsrichtung passiert, wobei die zweite kontinuierliche Lage mit einem zweiten Fördergeschwindigkeitswert (mpm), der höher als der erste Fördergeschwindigkeitswert (mpm) ist, zu der zweiten vorbestimmten Position gefördert wird,
wobei die Anzahl der Aufnahmeklingen in der Rotationsrichtung auf der Halterolle und die Anzahl der Schneideklingen in der Rotationsrichtung auf der Schneidrolle identisch sind,
wobei ein Anordnungsabstand in der Rotationsrichtung der Aufnahmeklinge auf einem mit der Aufnahmeklinge gezogenen Umlaufbahn aufgrund der Rotation der Halterolle in der Rotationsrichtung identisch ist mit einem Anordnungsabstand in der Rotationsrichtung der Schneideklinge auf einem mit der Schneideklinge gezogenen Umlaufbahn aufgrund der Rotation der Schneiderolle in der Rotationsrichtung, und
wobei die Halterolle auf der Grundlage eines definierten Geschwindigkeitsmusters derart rotiert, dass ein Geschwindigkeitswert (mpm) in der Rotationsrichtung der Aufnahmeklinge beim Passieren der zweiten vorbestimmten Position identisch mit dem zweiten Fördergeschwindigkeitswert ist, und derart, dass ein Geschwindigkeitswert (mpm) in der Rotationsrichtung der Aufnahmeklinge beim Passieren der ersten vorbestimmten Position niedriger ist als der Geschwindigkeitswert (mpm) in der Rotationsrichtung der Aufnahmeklinge beim Passieren der zweiten vorbestimmten Position.

## Revendications

1. Procédé de fabrication d'un corps composite de feuilles continues se rapportant à un article absorbant, dans lequel une première feuille continue est découpée pour produire une feuille découpée, et la feuille découpée est collée et transférée sur une seconde feuille continue tout en formant un intervalle sur la seconde feuille continue entre la feuille découpée et une autre feuille découpée qui lui est adjacente dans un sens de continuité de la seconde feuille continue, le procédé comprenant :
le transport de la première feuille continue à une première valeur de vitesse de transport (mpm) dans un sens de transport qui est un sens de continuité de la première feuille continue ;
la rotation d'un rouleau de maintien en utilisant un moteur dans un sens de rotation conforme au sens de transport de la première feuille continue, le rouleau de maintien étant mis en rotation tandis que la première feuille continue est maintenue sur une surface circonférentielle extérieure du rouleau de maintien de sorte que la première feuille continue coulisse en amont dans le sens de rotation sur la surface circonférentielle extérieure ;
la rotation d'un rouleau de coupe en utilisant un moteur séparé du moteur pour le rouleau de maintien, le rouleau de coupe étant agencé dans une première position prédéterminée dans le sens de rotation du rouleau de maintien et étant tourné dans un sens de rotation conforme au sens de transport ;
la découpe et la production de la feuille découpée avec une lame réceptrice dans la surface circonférentielle extérieure du rouleau de maintien coupant la première feuille continue conjointement avec une lame de coupe du rouleau de coupe lorsque la lame réceptrice passe la première position prédéterminée dans le sens de rotation du rouleau de maintien ; et
le transfert de la feuille découpée sur la surface circonférentielle extérieure sur la seconde feuille continue et le collage de la feuille découpée sur la seconde feuille continue lorsque la feuille découpée maintenue de manière non coulissante sur la surface circonférentielle extérieure passe une seconde position prédéterminée dans le sens de rotation, la seconde feuille continue étant transportée vers la seconde position prédéterminée à une seconde valeur de vitesse de transport (mpm) qui est supérieure à la première valeur de vitesse de transport (mpm),
dans lequel le nombre de lames réceptrices dans le sens de rotation sur le rouleau de maintien et le nombre de lames de coupe dans le sens de rotation sur le rouleau de coupe sont identiques,
un pas d'agencement dans le sens de rotation de la lame réceptrice sur un chemin de rotation dessiné avec la lame réceptrice en raison de la rotation du rouleau de maintien dans le sens de rotation est identique à un pas d'agencement dans le sens de rotation de la lame de coupe dans un chemin de rotation dessiné avec la lame de coupe en raison de la rotation du rouleau de coupe dans le sens de rotation,
le rouleau de maintien tourne sur la base d'une configuration de vitesse définie de sorte qu'une valeur de vitesse (mpm) dans le sens de rotation de la lame réceptrice lors du passage de la seconde position prédéterminée est identique à la seconde valeur de vitesse de transport, et de sorte qu'une valeur de vitesse (mpm) dans le sens de rotation de la lame réceptrice lors du passage de la première position prédéterminée est inférieure à la valeur de vitesse (mpm) dans le sens de rotation de la lame réceptrice lors du passage de la seconde position prédéterminée, et
lors de la découpe et de la production, la valeur de vitesse (mpm) dans le sens de rotation de la lame réceptrice lors du passage de la première position prédéterminée est supérieure à la première valeur de vitesse de transport de la première feuille continue.

2. Procédé de fabrication d'un corps composite de feuilles continues se rapportant à un article absorbant selon la revendication 1,
dans lequel la seconde valeur de vitesse de transport de la seconde feuille continue est maintenue constante, et
lors du transfert, la valeur de vitesse (mpm) dans le sens de rotation de la lame réceptrice est maintenue constante du début à la fin du transfert de la feuille découpée sur la seconde feuille continue.

3. Procédé de fabrication d'un corps composite de feuilles continues se rapportant à un article absorbant selon la revendication 1 ou 2, dans lequel la valeur de vitesse (mpm) dans le sens de rotation de la lame réceptrice lors du passage de la première position prédéterminée est supérieure à une moyenne arithmétique de la première valeur de vitesse de transport et de la seconde valeur de vitesse de transport.

4. Procédé de fabrication d'un corps composite de feuilles continues se rapportant à un article absorbant selon la revendication 1 ou 2, dans lequel la valeur de vitesse (mpm) dans le sens de rotation de la lame réceptrice lors du passage de la première position prédéterminée est inférieure à une moyenne arithmétique de la première valeur de vitesse de transport et de la seconde valeur de vitesse de transport.

5. Procédé de fabrication d'un corps composite de feuilles continues se rapportant à un article absorbant selon l'une quelconque des revendications 1 à 4,
dans lequel la lame de coupe dépasse d'une surface circonférentielle extérieure du rouleau de coupe, et
un adhésif pour le collage de la seconde feuille continue et de la feuille découpée est appliqué sur, parmi deux surfaces de la première feuille continue, une surface qui fait face à la lame de coupe.

6. Procédé de fabrication d'un corps composite de feuilles continues se rapportant à un article absorbant selon l'une quelconque des revendications 1 à 5, dans lequel lors de la découpe et de la production, la valeur de vitesse (mpm) dans le sens de rotation de la lame réceptrice est maintenue constante du début à la fin de la découpe de la première feuille continue avec la lame de coupe et la lame réceptrice.

7. Procédé de fabrication d'un corps composite de feuilles continues se rapportant à un article absorbant selon l'une quelconque des revendications 1 à 6, dans lequel la première valeur de vitesse de transport de la première feuille continue est maintenue constante.

8. Procédé de fabrication d'un corps composite de feuilles continues se rapportant à un article absorbant selon l'une quelconque des revendications 1 à 7,
dans lequel la lame réceptrice est un élément séparé du rouleau de maintien,
la lame réceptrice a une surface d'arc qui s'étend le long du sens de rotation du rouleau de maintien, et
la première feuille continue est serrée entre et coupée avec la surface d'arc et la lame de coupe.

9. Procédé de fabrication d'un corps composite de feuilles continues se rapportant à un article absorbant selon l'une quelconque des revendications 1 à 8,
dans lequel une pluralité de lames réceptrices et une pluralité de lames de coupe sont prévues, et
la seconde position prédéterminée est réglée dans une position dans le sens de rotation du rouleau de maintien qui est une position dans laquelle, lorsque l'une de la pluralité de lames réceptrices passe la première position prédéterminée, une autre des lames réceptrices n'est pas localisée.

10. Appareil de fabrication d'un corps composite de feuilles continues se rapportant à un article absorbant, dans lequel une première feuille continue est découpée pour produire une feuille découpée, et la feuille découpée est collée et transférée sur une seconde feuille continue tout en formant un intervalle sur la seconde feuille continue entre la feuille découpée et une autre feuille découpée qui lui est adjacente dans un sens de continuité de la seconde feuille continue, l'appareil comprenant :
un premier mécanisme de transport qui transporte la première feuille continue à une première valeur de vitesse de transport (mpm) dans un sens de transport qui est un sens de continuité de la première feuille continue ;
un mécanisme de rouleau de maintien ayant un rouleau de maintien qui est entraîné pour tourner en utilisant un moteur dans un sens de rotation conforme au sens de transport de la première feuille continue, le mécanisme de rouleau de maintien faisant tourner le rouleau de maintien tandis que la première feuille continue est maintenue sur une surface circonférentielle extérieure du rouleau de maintien de sorte que la première feuille continue glisse en amont dans le sens de rotation sur la surface circonférentielle extérieure ; et
un rouleau de coupe qui est agencé dans une première position prédéterminée dans le sens de rotation du rouleau de maintien, le rouleau de coupe étant entraîné pour tourner dans un sens de rotation conforme au sens de transport, en utilisant un moteur séparé du moteur pour le rouleau de maintien,
dans lequel la feuille découpée est produite par une lame réceptrice au niveau de la surface circonférentielle extérieure du rouleau de maintien coupant la première feuille continue conjointement avec une lame de coupe du rouleau de coupe, dans la valeur de vitesse (mpm) dans le sens de rotation supérieure à la première valeur de vitesse de transport de la première feuille continue, lorsque la lame réceptrice passe la première position prédéterminée dans le sens de rotation du rouleau de maintien,
la feuille découpée sur la surface circonférentielle extérieure est collée et transférée sur la seconde feuille continue lorsque la feuille découpée maintenue de manière non coulissante sur la surface circonférentielle extérieure passe une seconde position prédéterminée dans le sens de rotation, la seconde feuille continue étant transportée vers la seconde position prédéterminée à une seconde valeur de vitesse de transport (mpm) qui est supérieure à la première valeur de vitesse de transport (mpm),
le nombre de lames réceptrices dans le sens de rotation sur le rouleau de maintien et le nombre de lames de coupe dans le sens de rotation sur le rouleau de coupe sont identiques,
un pas d'agencement dans le sens de rotation de la lame réceptrice sur un chemin de rotation dessiné avec la lame réceptrice en raison de la rotation du rouleau de maintien dans le sens de rotation est identique à un pas d'agencement dans le sens de rotation de la lame de coupe dans un chemin de rotation dessiné avec la lame de coupe en raison de la rotation du rouleau de coupe dans le sens de rotation, et
le rouleau de maintien tourne sur la base d'une configuration de vitesse définie de sorte qu'une valeur de vitesse (mpm) dans le sens de rotation de la lame réceptrice lors du passage de la seconde position prédéterminée est identique à la seconde valeur de vitesse de transport, et de sorte qu'une valeur de vitesse (mpm) dans le sens de rotation de la lame réceptrice lors du passage de la première position prédéterminée est inférieure à la valeur de vitesse (mpm) dans le sens de rotation de la lame réceptrice lors du passage de la seconde position prédéterminée.
